# EUROPEAN PATENT APPLICATION

(11) **EP 4 115 887 A1**
(43) Date of publication of application: **11.01.2023**
(21) Application number: 21764686.8
(22) Date of filing: 03.03.2021
(51) Int. Cl.: A61K 31/5355, A61K 31/444, A61K 31/4365, A61P 35/00

(54) **PHARMACEUTICAL COMPOSITION FOR PREVENTION OR TREATMENT OF CANCER IN WHICH KRAS MUTATION AND ACTIVATED RON ARE PRESENT**

(30) Priority: 03.03.2020 KR 20200026381
(71) Applicant: Wellmarker Bio Co., Ltd., Seoul 05855 (KR)
(72) Inventor: SHIN, Jae-Sik, Seoul 05855 (KR); KO, Young-Ok, Seoul 05855 (KR); LEE, Min-Ki, Seoul 05855 (KR); KIM, Yong-Seok, Seoul 05855 (KR); CHOI, Soon-Jin, Seoul 05855 (KR); LIM, Na-Jung, Seoul 05855 (KR); KIM, Min-Hwa, Seoul 05855 (KR); LEE, Jun-Hyung, Seoul 05855 (KR)
(74) Representative: HGF
(86) International application number: PCT/KR2021/002622
(87) International publication number: WO 2021/177721

(57) **Abstract**

The present invention relates to a pharmaceutical composition for preventing or treating cancer with a KRAS mutation and activated RON. Specifically, the pharmaceutical composition may be utilized as an anticancer drug that exhibits an excellent cell death-inducing ability on cancer cells with KRAS G13 mutation or KRAS G12 mutation and a RON mutation or tyrosine-phosphorylated RON (pTyr-RON).

## Description

### Technical Field

The present invention relates to a pharmaceutical composition for preventing or treating cancer which is applicable to a cancer patient having cancer cells with a KRAS mutation and activated RON.

### Background Art

A V-Ki-ras2 Kirsten rat sarcoma viral oncogene homolog (KRAS) gene is one of several genes associated with the development of non-small cell lung cancer, colorectal cancer, pancreatic cancer, and the like, and has been considered crucial to determine the effectiveness of an anticancer drug such as cetuximab. Specifically, in consideration of a mutation of the KRAS gene, the KRAS gene is known to significantly improve drug response and survival in cancer patients.

On the other hand, recepteur d'origine nantais (RON) refers to a protein receptor belonging to the c-MET family, and is a receptor for macrophage-stimulating protein (MSP), a serum protein, which is secreted by the liver and regulates the action of macrophages. The activity of RON plays an important role in the development, progression, and metastasis of tumors. In particular, overexpression or hyperactivity thereof in colorectal cancer and breast cancer has been reported to contribute to inducing tumor invasion and metastasis and inhibiting cell death. Substances capable of specifically inhibiting such abnormal activity of RON can effectively treat various diseases related to RON, in particular, tumors such as colorectal cancer.

Therefore, studies on substances, which are capable of specifically killing cancer patient's cancer cells with a KRAS mutation and abnormally activated RON, have recently been continuously and actively conducted.

### [PRIOR ART DOCUMENTS]

(Non-patent document 1) Targeting Receptor Kinases in Colorectal Cancer, Cancers (2019), 11, 433-456.
(Non-patent document 2) Comprehensive review of targeted therapy for colorectal cancer, Sig Transduct Target Ther (2020), 5, 22-51
(Non-patent document 3) New Strategies for Treatment of KRAS Mutant Metastatic Colorectal Cancer, Clin Cancer Res., (2010), 16, 2921-2926.

### Disclosure of Invention

### Technical Problem

The present inventors have tried to develop a pharmaceutical composition for preventing or treating cancer which is applicable to cancer patients having a KRAS mutation and abnormally activated RON. As a result, the present inventors have identified that compounds having a specific structure exhibit an excellent cell-killing effect on cancer cells with a KRAS mutation and activated RON, and thus have completed the present invention.

An object of the present invention is to provide a pharmaceutical composition for preventing or treating cancer with a KRAS mutation and activated RON.

### Solution to Problem

In order to achieve the above object, there is provided a pharmaceutical composition for preventing or treating cancer with a KRAS mutation and activated RON, comprising, as an active ingredient, a compound represented by Formula 1 or 2, or a pharmaceutically acceptable salt thereof, wherein the KRAS mutation is substitution of glycine, an amino acid residue, at position 13 in the amino acid sequence of SEQ ID NO: 1 with another amino acid, or substitution of glycine, an amino acid residue, at position 12 with another amino acid.

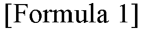

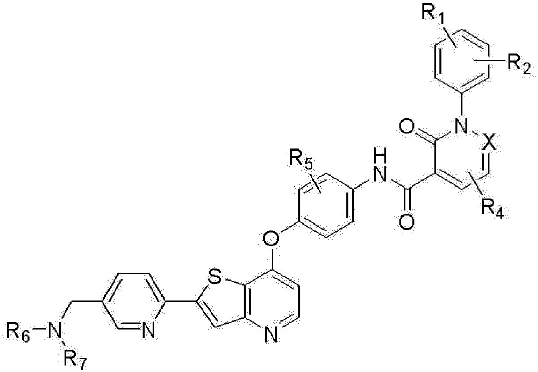

In Formula 1 above, R₁ to R₇, and X are the same as defined in the detailed description which will be described later.

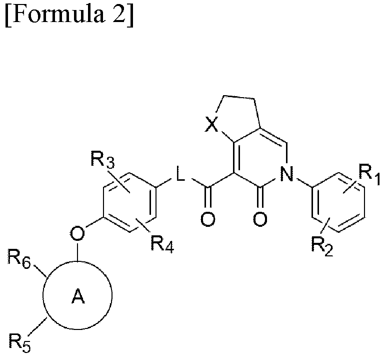

In Formula 2 above, R₁ to R₆, L and X are the same as defined in the detailed description which will be described later.

### Advantageous Effects of Invention

The pharmaceutical composition according to the present invention is applicable to cancer patients having a KRAS mutation and activated RON. Specifically, the pharmaceutical composition may be utilized as an anticancer drug that exhibits an excellent cell death-inducing ability on cancer cells with KRAS G13 mutation or KRAS G12 mutation and a RON mutation (Δ155, Δ160, Δ165) or tyrosine-phosphorylated RON (pTyr-RON).

### Brief Description of Drawings

FIG. 1 shows results obtained by identifying cell death induction efficacy of compounds of Example 1 and positive control 1 in a colorectal cancer cell line with a KRAS mutation (G12V or G13D) and activated RON.
FIG. 2 shows a cell death rate after SW620 cell line (KRAS G12V/mRON Δ155) is treated with a compound of Example 1, positive control 1, positive control 2, or positive control 3.
FIG. 3 shows results obtained by identifying, through immunofluorescence staining, the expression of pTyr-RON in various colorectal cancer patient-derived cell lines.
FIG. 4 shows results obtained by subjecting various colorectal patient-derived cell lines to treatment with the compound of Example 1 and comparing cell death induction efficacy thereof depending on the presence or absence of a KRAS mutation and activated RON.
FIG. 5 shows changes in tumor volume observed during 4-week oral administration of the compound of Example 1 to a mouse model transplanted with the cell line SW620.
FIG. 6 shows tumor growth inhibition observed by measuring a tumor weight after 4-week oral administration of the compound of Example 1 to a mouse model transplanted with the cell line SW620.
FIG. 7 shows results obtained by identifying, through immunohistochemistry, changes in expression of proteins after 4-week oral administration of the compound of Example 1 to a mouse model transplanted with the cell line SW620.
FIG. 8 shows a difference in relative tumor volume observed after 4-week oral administration of the respective compounds of Example 1, positive control 1, and positive control 2 to a mouse model transplanted with the cell line HTC8 (KRAS G13 D/pTyr-wtRON).
FIG. 9 shows results obtained by analyzing changes in expression of pTyr-mRON, mRON, cleaved caspase 3, and cyclin D1 in tumor tissues after 4-week oral administration of the respective compounds of Example 1, positive control 1, and positive control 2 to animals transplanted with the cell line HTC8.
FIG. 10 shows results obtained by causing the cell line SW620 to overexpress β-catenin, performing treatment with the compound of Example 1, and analyzing an effect thereof on cell death rate.
FIG. 11 shows results obtained by causing the cell line SW620 to overexpress β-catenin, performing treatment with the compound of Example 1, and then identifying, with the western blotting method, changes in expression of respective proteins.
FIG. 12 shows changes in tumor volume observed during 4-week oral administration of the compound of Example 1 to animals transplanted with the cell line HCT15 (KRAS G13D/mRON Δ160).
FIG. 13 shows results obtained by analyzing changes in expression of pTyr-mRON, mRON, cleaved caspase 3, and cyclin D1 in tumor tissues after 4-week oral administration of the compound of Example 1 to animals transplanted with the cell line HTC15.

### Best Mode for Carrying out the Invention

Hereinafter, the present invention will be described in detail.

In an aspect of the present invention, there is provided a pharmaceutical composition for preventing or treating cancer with a KRAS mutation and activated RON, the pharmaceutical composition comprising, as an active ingredient, a compound represented by Formula 1 or 2 or a pharmaceutically acceptable salt thereof, wherein the KRAS mutation is substitution of glycine, an amino acid residue, at position 13 in the amino acid sequence of SEQ ID NO: 1 with another amino acid, or substitution of glycine, an amino acid residue, at position 12 with another amino acid.

As used herein, the term "KRAS" is also called K-Ras and refers to a factor that constitutes part of RAS/MAPK, a cell signaling pathway that regulates cell growth, cell maturation, and cell death. Mutated versions of the KRAS are found in various types of solid cancers such as non-small cell lung cancer, colorectal cancer, and pancreatic cancer. In this case, the KRAS may have the amino acid sequence of SEQ ID NO: 1.

As used herein, the term "KRAS mutation" means that substitution, deletion, or insertion has occurred in some amino acids of KRAS. For example, the KRAS mutation may mean that the 12^{th} or 13^{th} amino acid in SEQ ID NO: 1 is substituted with another amino acid. Specifically, the KRAS mutation may be substitution of the glycine at position 12 with any one selected from the group consisting of aspartic acid, valine, cysteine, and histidine. Alternatively, the KRAS mutation may be substitution of the glycine at position 13 with any one selected from the group consisting of aspartic acid, valine, and arginine. More specifically, the KRAS mutation may feature that glycine, an amino acid residue, at position 13 is substituted with aspartic acid, or glycine, an amino acid residue, at position 12 is substituted with valine or histidine.

As used herein, the term "activated RON" may be understood as a concept including all RON's that can be identified as active at any expression level such as DNA level, mRNA level, and protein level. Specifically, the activated RON may be caused by phosphorylation in the RON protein, for example, phosphorylation in the kinase domain of the RON protein, and may be induced in the presence of a ligand such as MSP. In addition, the activated RON may include splicing variants or mutants of RON gene which are always in an activated form even without any ligand.

The activated RON may be a splicing variant of RON gene or may be tyrosine-phosphorylated RON protein. The splicing variant of RON gene may be caused by deletion, due to splicing, of at least one selected from the group consisting of exons 5, 6, and 11. Specifically, such a splicing variant may be mRONΔ155 in which exons 5, 6, and 11 are deleted, mRONΔ160 in which exons 5 and 6 are deleted, or RONΔ165 in which exon 11 is deleted. In addition, cDNA of mRONΔ155 may have the nucleotide sequence of SEQ ID NO: 2; cDNA of mRONΔ160 may have the nucleotide sequence of SEQ ID NO: 3; and cDNA of RONΔ165 may have the nucleotide sequence of SEQ ID NO: 4.

In the present invention, the cancer may be selected from, but is not limited to, the group consisting of breast cancer, lung cancer, gastric cancer, prostate cancer, uterine cancer, ovarian cancer, kidney cancer, pancreatic cancer, liver cancer, colorectal cancer, skin cancer, head and neck cancer, and thyroid cancer.

In another aspect of the present invention, there is provided a use of the compound represented by Formula 1 or 2 or a pharmaceutically acceptable salt thereof for preventing or treating cancer with the KRAS mutation and activated RON.

In yet another aspect of the present invention, there is provided a use of the compound represented by Formula 1 or 2 or a pharmaceutically acceptable salt thereof for preparation of a medicament for preventing or treating cancer with the KRAS mutation and activated RON.

In still yet another aspect of the present invention, there is provided a method for preventing or treating cancer with the KRAS mutation and activated RON, the method comprising administering the compound represented by Formula 1 or 2 or a pharmaceutically acceptable salt thereof to a subject in need thereof.

In another aspect of the present invention, there is provided a method for treating a cancer patient who carries a KRAS mutation and activated RON, comprising: identifying whether the patient carries G13D, G13V, G13R, G12V, G12D, G12C, or G12H mutation in KRAS and carries activated RON; and administering, to the patient who carries the KRAS mutation and activated RON, an anticancer drug that comprises, as an active ingredient, the compound represented by Formula 1 or 2 or a pharmaceutically acceptable salt thereof.

In yet another aspect of the present invention, there is provided a method for providing information on an anticancer therapeutic agent, the method comprising: identifying a KRAS mutation and activated RON in an individual; and providing information that the compound represented by Formula 1 or 2 or a pharmaceutically acceptable salt thereof is suitable for anticancer therapy of the individual when activated RON and a KRAS mutation in which glycine, an amino acid residue, at position 13 in the amino acid sequence of SEQ ID NO: 1 is substituted with another amino acid or glycine, an amino acid residue, at position 12 is substituted with another amino acid, are found.

In the present invention, the "prevention" refers to any act of inhibiting or delaying occurrence, spread, and recurrence of the disease; and the "treatment" refers to any act of ameliorating or beneficially altering symptoms of the disease by administration of the compound.

In addition, the term "subject" or "individual" refers to any animal, specifically a mammal, such as a monkey, a cow, a horse, a sheep, a pig, a chicken, a turkey, a quail, a cat, a dog, a mouse, a rat, a rabbit, and a guinea pig, including a human (patient), who has or is likely to develop the disease related to protein kinase activity. In addition, the subject in need thereof may mean a biological sample.

In addition, the "administration" means providing a predetermined substance to a subject in need thereof by any appropriate method, and administration of the compound of the present invention may be achieved via any common route as long as the route allows the substance to reach a target tissue.

The compound of Formula 1 used in the present invention is represented as below:

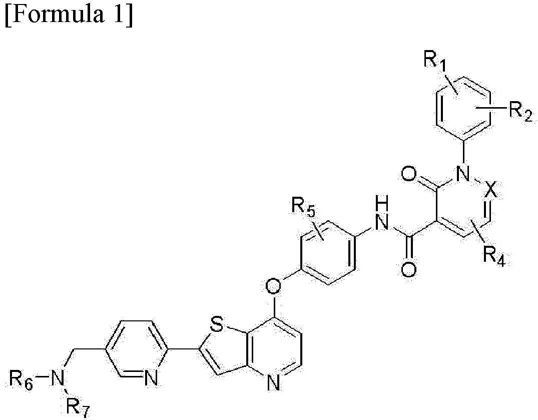

In Formula 1 above,
R₁ and R₂ are each independently H, halogen, C₁₋₁₀ alkoxy, or C₁₋₁₀ haloalkyl;
X is -C(-R₃)= or -N=;
R₃ and R₄ are each independently H, halogen, C₁₋₁₀ alkyl, or C₁₋₁₀ alkoxy;
R₅ is H, halogen, or C₁₋₁₀ alkyl;
R₆ and R₇ form a 4- to 10-membered heterocycle together with the N atom to which they are bonded, or R₆ is -C₂H₄-O-CH₃, and R₇ is H, methyl, or t-butoxycarbonyl; and
the heterocycle optionally further contain one or two heteroatoms selected from the group consisting of N, O, and S, in addition to the N atom to which R₆ and R₇ are bonded, and is unsubstituted or substituted with one or more substituents selected from halogen and C₁₋₆ alkyl.

The C₁₋₁₀ alkyl may include C₁₋₆ alkyl, C₁₋₃ alkyl, C₃₋₁₀ alkyl, C₃₋₆ alkyl, C₆₋₁₀ alkyl, and the like. In addition, the C₁₋₁₀ alkoxy may include C₁₋₆ alkoxy, C₁₋₃ alkoxy, C₃₋₁₀ alkoxy, C₃₋₆ alkoxy, C₆₋₁₀ alkoxy, and the like. In addition, the 4- to 10-membered heterocycle may include 4- to 7-membered heterocycle, 4- to 6-membered heterocycle, 5- to 7-membered heterocycle, 5- or 6-membered heterocycle, and the like.

According to one embodiment, in Formula 1, R₁ and R₂ are each independently H, halogen, methoxy, or -CF₃, wherein the halogen may be F, Cl, Br, or I.

According to another embodiment, in Formula 1, R₃ and R₄ are each independently H, halogen, methyl, methoxy, or ethoxy, wherein the halogen may be F, Cl, Br, or I.

According to a further embodiment, in Formula 1, X is -C(-R₃)=; and R₃ and R₄ are each independently H, halogen, methyl, methoxy, or ethoxy, but not simultaneously H.

According to a still further embodiment, in Formula 1, X is -N=; and R₄ is halogen, methyl, methoxy, or ethoxy, wherein the halogen may be F, Cl, Br, or I.

According to a still further embodiment, in Formula 1, R₅ is H or halogen, wherein the halogen may be F, Cl, Br, or I.

According to a still further embodiment, in Formula 1, R₆ and R₇, taken together with the N atom to which they are bonded, form 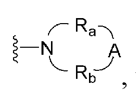 , wherein Rₐ and R_{b} are each independently C₁₋₃ alkylene, A is -N(-R₉)- or -O-, and R₉ is C₁₋₆ alkyl. As specific examples, R₆ and R₇, together with the N atom to which they are bonded, may form a heterocycle group such as azetidinyl, diazetidinyl, pyrrolidinyl, pyrrolyl, imidazolidinyl, imidazolyl, pyrazolidinyl, pyrazolyl, oxazolidinyl, oxazolyl, isoxazolidinyl, isoxazolyl, thiazolidinyl, thiazolyl, isothiazolidinyl, isothiazolyl, piperidinyl, pyridinyl, piperazinyl, diazinyl, morpholino, thiomorpholino, azepanyl, and diazepanyl, which is optionally substituted with C₁₋₆ alkyl. Further, Rₐ and R_{b} may each independently be -CH₂-, -C₂H₄-, or -C₃H₆-. In addition, R₉ may be methyl, ethyl, n-propyl, i-propyl, n-butyl, i-butyl, t-butyl, n-pentyl, i-pentyl, t-pentyl, secpentyl, neopentyl, hexyl, and the like..

According to a still further embodiment, in Formula 1, R₁ and R₂ are each independently H, halogen, methoxy, or -CF₃; R₃ and R₄ are each independently H, halogen, methyl, methoxy, or ethoxy; R₅ is H or halogen; and R₅ is -C₂H₄-O-CH₃ and R₇ is H, methyl, or t-butoxycarbonyl, or R₆ and R₇ are bonded together to form a morpholino or methylpiperazinyl group. Said halogen may be F, Cl, Br, or I.

According to one embodiment, the compound of Formula 1 may be represented by Formula 1a below. 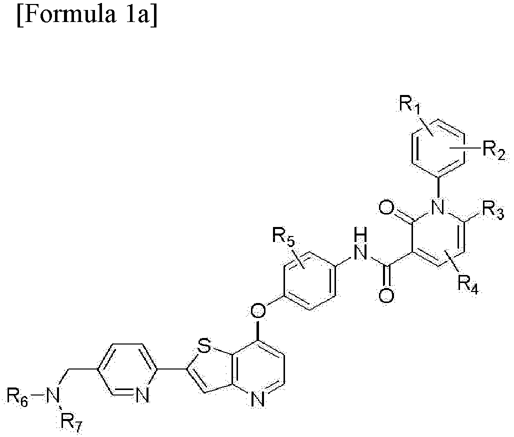 wherein R₁ to R₇ are the same as defined in the above Formula 1.

Specifically, in the above Formula 1a, R₁ and R₂ may each independently be H, halogen, or -CF₃. Further, R₃ and R₄ may each independently be H, halogen, methyl, methoxy, or ethoxy, but are not simultaneously H. In addition, R₅ may be H or halogen.

More specifically, in the above Formula 1a, R₁ and R₂ may each independently be H, halogen, or -CF₃; R₃ and R₄ may each independently be H, halogen, methyl, methoxy, or ethoxy; R₅ may be H or halogen; R₆ may be -C₂H₄-O-CH₃; and R₇ may be H, methyl, or t-butoxycarbonyl.

According to another embodiment, the compound of Formula 1 may be represented by Formula 1b below. 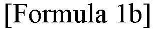 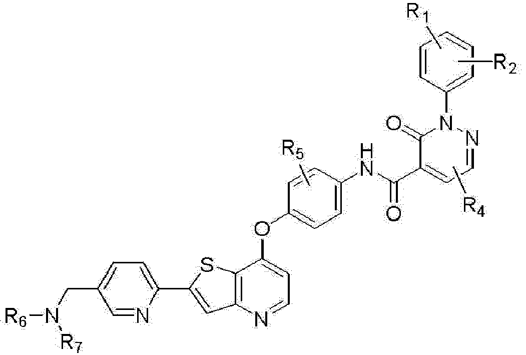 wherein R₁ to R₇ are the same as defined in the above Formula 1.

Specifically, in the above Formula 1b, R₁ and R₂ may each independently be H, halogen, or -CF₃. Further, R₄ may be halogen, methyl, methoxy, or ethoxy. In addition, R₅ may be H or halogen.

More specifically, in the above Formula 1b, R₁ and R₂ may each independently be H, halogen, or -CF₃; R₄ may be halogen, methyl, methoxy, or ethoxy; R₅ may be H or halogen; R₆ may be -C₂H₄-O-CH₃; and R₇ may be H, methyl, or t-butoxycarbonyl.

According to a further embodiment, the compound of Formula 1 may be represented by Formula 1c below. 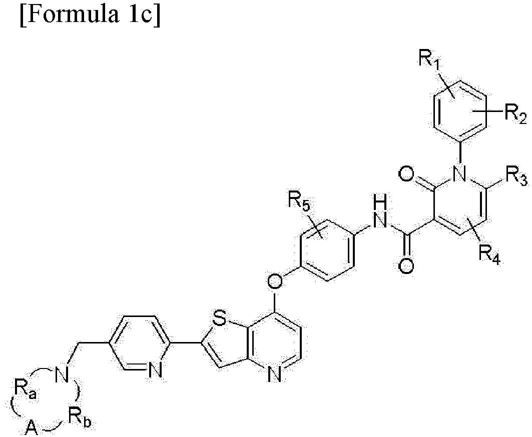 wherein R₁ to R₅ are the same as defined in the above Formula 1; Rₐ and R_{b} may each independently be C₁₋₃ alkylene; A may be -N(-R₉)-, or -O-; and R₉ may be C₁₋₆ alkyl.

Specifically, in the above Formula 1c, R₁ and R₂ may each independently be H, halogen, or -CF₃. Further, R₃ and R₄ may each independently be H, halogen, methyl, methoxy, or ethoxy, but are not simultaneously H. In addition, R₅ may be H or halogen. Further, Rₐ and R_{b} may form a morpholino or methylpiperazinyl group together with N and A to which they are bonded.

According to a still further embodiment, the compound of Formula 1 may be represented by Formula Id below. 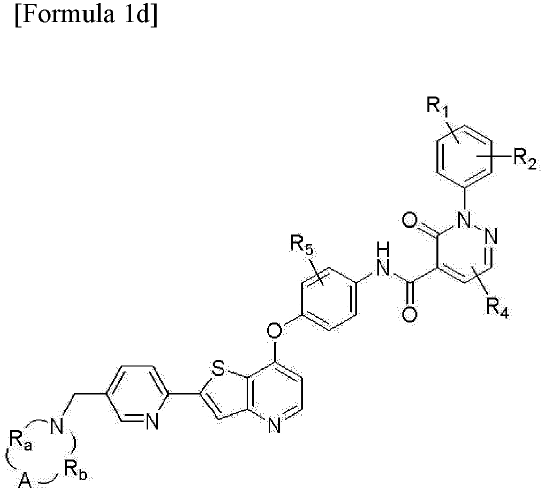 wherein R₁ to R₅ are the same as defined in the above Formula 1; Rₐ and R_{b} may each independently be C₁₋₃ alkylene; A may be -N(-R₉)- or -O-; and R₉ may be C₁₋₆ alkyl.

Specifically, in the above Formula Id, R₁ and R₂ may each independently be H, halogen, or -CF₃. Further, R₄ may be halogen, methyl, methoxy, or ethoxy. In addition, R₅ may be H or halogen. Further, Rₐ and R_{b} may form a morpholino or methylpiperazinyl group together with N and A to which they are bonded.

Specific examples of the compound of Formula 1 are listed below:
a1) 4-ethoxy-N-(3-fluoro-4-{[2-(5-{[(2-methoxyethyl)amino]methyl}pyridin-2-yl)thieno[3,2-b]pyridin-7-yl]oxy}phenyl)-2-oxo-1-phenyl-1,2-dihydropyridine-3-carboxamide;
a2) N-(3-fluoro-4-{ [2-(5-{ [(2-methoxyethyl)amino]methyl}pyridin-2-yl)thieno[3,2-b]pyridin-7-yl]oxy}phenyl)-1-(4-fluorophenyl)-4-methoxy-2-oxo-1,2-dihydropyridine-3 -carboxamide;
a3) N-(3-fluoro-4-{ [2-(5-{ [(2-methoxyethyl)amino]methyl}pyridin-2-yl)thieno[3,2-b]pyridin-7-yl]oxy}phenyl)-4-methoxy-2-oxo-1-phenyl-1,2-dihydropyridine-3 -carboxamide;
a4) N-(3-fluoro-4-{ [2-(5-{ [(2-methoxyethyl)amino]methyl}pyridin-2-yl)thieno[3,2-b]pyridin-7-yl]oxy}phenyl)-1-(4-fluorophenyl)-2-oxo-1,2-dihydropyridine-3 -carboxamide;
a5) N-(3-fluoro-4-{[2-(5-{[(2-methoxyethyl)amino]methyl}pyridin-2-yl)thieno[3,2-b]pyridin-7-yl]oxy}phenyl)-2-oxo-1-phenyl-1,2-dihydropyridine-3-carboxamide;
a6) t-Butyl {[6-(7-{4-[4-ethoxy-1-(4-fluorophenyl)-2-oxo-1,2-dihydropyridine-3-carboxamido]-2-fluorophenoxy}thieno[3,2-b]pyridin-2-yl)pyridin-3 -yl]methyl} (2-methoxyethyl)carbamate;
a7) 4-ethoxy-N-(3-fluoro-4-{[2-(5-{[(2-methoxyethyl)amino]methyl}pyridin-2-yl)thieno[3,2-b]pyridin-7-yl]oxy}phenyl)-1-(4-fluorophenyl)-2-oxo-1,2-dihydropyridine-3 -carboxamide;
a8) 1-(4-chlorophenyl)-4-ethoxy-N-(3-fluoro-4-{[2-(5-{[(2-methoxyethyl)amino]methyl}pyridin-2-yl)thieno[3,2-b]pyridin-7-yl]oxy}phenyl)-2-oxo-1,2-dihydropyridine-3 -carboxamide;
a9) N-(3-chloro-4-{[2-(5-{[(2-methoxyethyl)amino]methyl}pyridin-2-yl)thieno[3,2-b]pyridin-7-yl]oxy}phenyl)-1-(4-fluorophenyl)-4-methoxy-2-oxo-1,2-dihydropyridine-3 -carboxamide;
a10) N-(2-chloro-4-{[-2-(5-{[(2-methoxyethyl)amino]methyl}pyridin-2-yl)thieno[3,2-b]pyridin-7-yl]oxy}phenyl)-1-(4-fluorophenyl)-4-methoxy-2-oxo-1,2-dihydropyridine-3 -carboxamide;
a11) 1-(4-fluorophenyl)-4-methoxy-N-(4-{[2-(5-{[(2-methoxyethyl)amino]methyl}pyridin-2-yl)thieno[3,2-b]pyridin-7-yl)oxy)phenyl)-2-oxo-1,2-dihydropyridine-3 -carboxamide;
a12) 4-ethoxy-N-(3-fluoro-4-{[2-(5-{[(2-methoxyethyl)amino]methyl}pyridin-2-yl)thieno[3,2-b]pyridin-7-yl]oxy}phenyl)-2-oxo-1-(4-(trifluoromethyl)phenyl)-1,2-dihydropyridine-3-carboxamide;
a13) 1-(4-chlorophenyl)-N-(3-fluoro-4-{[2-(5-{[(2-methoxyethyl)amino]methyl}pyridin-2-yl)thieno[3,2-b]pyridin-7-yl]oxy}phenyl)-4-methoxy-2-oxo-1,2-dihydropyridine-3-carboxamide;
a14) 4-ethoxy-N-(3-fluoro-4-{[2-(5-{[(2-methoxyethyl)amino]methyl}pyridin-2-yl)thieno[3,2-b]pyridin-7-yl]oxy}phenyl)-1-(3-fluorophenyl)-2-oxo-1,2-dihydropyridine-3-carboxamide;
a15) 4-ethoxy-N-(3-fluoro-4-{[2-(5-{[(2-methoxyethyl)amino]methyl}pyridin-2-yl)thieno[3,2-b]pyridin-7-yl]oxy}phenyl)-1-(4-methoxyphenyl)-2-oxo-1,2-dihydropyridine-3 -carboxamide;
a16) 4-ethoxy-N-(3-fluoro-4-{[2-(5-{[(2-methoxyethyl)amino]methyl}pyridin-2-yl)thieno[3,2-b]pyridin-7-yl]oxy}phenyl)-1-(3-methoxyphenyl)-2-oxo-1,2-dihydropyridine-3-carboxamide;
a17) N-(3-fluoro-4-{ [2-(5-{ [(2-methoxyethyl)amino]methyl}pyridin-2-yl)thieno[3,2-b]pyridin-7-yl]oxy}phenyl)-2-(4-fluorophenyl)-5-methyl-3-oxo-2,3-dihydropyridazine-4-carboxamide;
a18) N-(3-fluoro-4-{[2-(5-{[(2-methoxyethyl)amino]methyl}pyridin-2-yl)thieno[3,2-b]pyridin-7-yl]oxy}phenyl)-5-methyl-3-oxo-2-phenyl-2,3-dihydropyridazine-4-carboxamide;
a19) N-(3-fluoro-4-{[2-(5-{ [(2-methoxyethyl)amino]methyl}pyridin-2-yl)thieno[3,2-b]pyridin-7-yl]oxylphenyl)-3-oxo-2-phenyl-2,3-dihydropyridazine-4-carboxamide;
a20) N-(3-fluoro-4-[{2-(5-[{(2-methoxyethyl)amino}methyl]pyridin-2-yl)thieno[3,2-b]pyridin-7-yl}oxy]phenyl)-2-(4-fluorophenyl)-3-oxo-2,3-dihydropyridazine-4-carboxamide;
a21) N-(3-fluoro-4-{[2-(5-{[(2-methoxyethyl)amino]methyl}pyridin-2-yl)thieno[3,2-b]pyridin-7-yl]oxy}phenyl)-6-methyl-2-oxo-1-phenyl-1,2-dihydropyridine-3 -carboxamide;
a22) N-(3-fluoro-4-{[2-(5-{[(2-methoxyethyl)amino]methyl}pyridin-2-yl)thieno[3,2-b]pyridin-7-yl]oxy}phenyl)-1-(4-fluorophenyl)-6-methyl-2-oxo-1,2-dihydropyridine-3 -carboxamide;
a23) 5-Bromo-N-(3-fluoro-4-{[2-(5-{[(2-methoxyethyl)amino]methyl}pyridin-2-yl)thieno[3,2-b]pyridin-7-yl]oxy}phenyl)-1-(4-fluorophenyl)-2-oxo-1,2-dihydropyridine-3 -carboxamide;
a24) 5-Chloro-N-(3-fluoro-4-{[2-(5-{[(2-methoxyethyl)amino]methyl}pyridin-2-yl)thieno[3,2-b]pyridin-7-yl]oxy}phenyl)-1-(4-fluorophenyl)-2-oxo-1,2-dihydropyridine-3-carboxamide;
a25) N-(3-fluoro-4-{[2-(5-{[(2-methoxyethyl)amino]methyl}pyridin-2-yl)thieno[3,2-b]pyridin-7-yl]oxy}phenyl)-1-(4-fluorophenyl)-4-methyl-2-oxo-1,2-dihydropyridine-3 -carboxamide;
a26) N-(2-chloro-4-{[2-(5-{[(2-methoxyethyl)amino]methyl}pyridin-2-yl)thieno[3,2-b]pyridin-7-yl]oxy}phenyl)-4-ethoxy-1-(4-fluorophenyl)-2-oxo-1,2-dihydropyridine-3 -carboxamide;
a27) N-(3-fluoro-4-([2-(5-{[(2-methoxyethyl)amino)methyl]pyridin-2-yl}thieno[3,2-b]pyridin-7-yl)oxy]phenyl}-1-(4-fluorophenyl)-5,6-dimethyl-2-oxo-1,2-dihydropyridine-3-carboxamide;
a28) N-(3-fluoro-4-{[-2-(5-{[(2-methoxyethyl)amino]methyl}pyridin-2-yl)thieno[3,2-b]pyridin-7-yl]oxy}phenyl)-4-methyl-2-oxo-1-phenyl-1,2-dihydropyridine-3 -carboxamide;
a29) N-(3-fluoro-4-{[-2-(5-{[(2-methoxyethyl)amino]methyl}pyridin-2-yl)thieno[3,2-b]pyridin-7-yl]oxy}phenyl)-1-(4-fluorophenyl)-5-methyl-2-oxo-1,2-dihydropyridine-3 -carboxamide;
a30) 4-Ethoxy-N-(3-fluoro-4-{[2-(5-{[(2-methoxyethyl)(methyl)amino]methyl}pyridin-2-yl)thieno[3,2-b]pyridin-7-yl]oxy}phenyl)-1-(4-fluorophenyl)-2-oxo-1,2-dihydropyridine-3-carboxamide;
a31) 4-ethoxy-N-[3-fluoro-4-({2-[5-(morpholinomethyl)pyridin-2-yl]thieno[3,2-b]pyridin-7-yl}oxy)phenyl]-1-(4-fluorophenyl)-2-oxo-1,2-dihydropyridine-3 -carboxamide;
a32) 4-ethoxy-N-[3-fluoro-4-({2-[5-(morpholinomethyl)pyridin-2-yl]thieno[3,2-b]pyridin-7-yl}oxy)phenyl]-2-oxo-1-phenyl-1,2-dihydropyridine-3-carboxamide;
a33) 4-ethoxy-N-{3-fluoro-4-[(2-{5-[(4-methylpiperazin-1-yl)methyl]pyridin-2-yl}thieno[3,2-b]pyridin-7-yl)oxy]phenyl}-1-(4-fluorophenyl)-2-oxo-1,2-dihydropyridine-3 -carboxamide;
a34) 4-ethoxy-N-{3-fluoro-4-[(2-{5-[(4-methylpiperazin-1-yl)methyl]pyridin-2-yl}thieno[3,2-b]pyridin-7-yl)oxy]phenyl}-2-oxo-1-phenyl-1,2-dihydropyridine-3-carboxamide;
a35) 1-(4-chlorophenyl)-4-ethoxy-N-[3-fluoro-4-({2-[5-(morpholinomethyl)pyridin-2-yl]thieno[3,2-b]pyridin-7-yl}oxy)phenyl]-2-oxo-1,2-dihydropyridine-3 -carboxamide;
a36) N-[3-chloro-4-({2-[5-(morpholinomethyl)pyridin-2-yl]thieno[3,2-b]pyridin-7-yl}oxy)phenyl]-4-ethoxy-1-(4-fluorophenyl)-2-oxo-1,2-dihydropyridine-3-carboxamide; and
a37) N-[2-chloro-4-({2-[5-(morpholinomethyl)pyridin-2-yl]thieno[3,2-b]pyridin-7-yl}oxy)phenyl]-4-ethoxy-1-(4-fluorophenyl)-2-oxo-1,2-dihydropyridine-3-carboxamide.

Preferably, the compounds of Formula 1 may be selected from the group consisting of:
4-ethoxy-N-[3-fluoro-4-({2-[5-(morpholinomethyl)pyridin-2-yl]thieno[3,2-b]pyridin-7-yl}oxy)phenyl]-1-(4-fluorophenyl)-2-oxo-1,2-dihydropyridine-3-carboxamide;
4-ethoxy-N-(3-fluoro-4-{[2-(5-{[(2-methoxyethyl)amino]methyl}pyridin-2-yl)thieno[3,2-b]pyridin-7-yl]oxy}phenyl)-2-oxo-1-phenyl-1,2-dihydropyridine-3-carboxamide; and
4-ethoxy-N-{3-fluoro-4-[(2-{5-[(4-methylpiperazin-1-yl)methyl]pyridin-2-yl}thieno[3,2-b]pyridin-7-yl)oxy]phenyl}-2-oxo-1-phenyl-1,2-dihydropyridine-3-carboxamide.

The compound of Formula 1 above used in the composition according to the present invention may be prepared by a method disclosed in Korean Patent Laid-open Publication No. 2019-0106802, and by other known methods and/or various methods based on the technology in the field of organic synthesis. Based on the above methods, various derivatives may be synthesized using an appropriate synthesis method according to the type of substituent.

The compound of Formula 2 used in the present invention is represented as below: 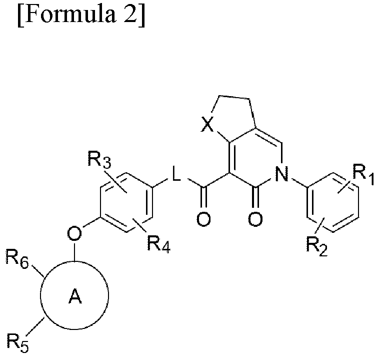 in the formula,
L is -NH- or -CH₂-,
R₁ to R₄ are each independently hydrogen, halogen, hydroxy, cyano, C₁₋₄ alkyl, C₁₋₄ alkoxy, C₁₋₄ haloalkyl, C₂₋₄ alkenyl, C₂₋₄ alkynyl, C₃₋₇ cycloalkyl, C₆₋₁₀ aryl, 5- to 9-membered heteroaryl, or 3- to 9-membered heterocycloalkyl,
X is O, S, -CH(-Rx)-, or -N(-Rx)-,
Rx is hydrogen, C₁₋₄ alkyl, C₁₋₄ alkoxy, C₁₋₄ haloalkyl, C₂₋₄ alkenyl, C₂₋₄ alkynyl, C₆₋₁₀ aryl, C₆₋₁₀ aryl-C₁₋₄ alkyl, or 3- to 9-membered heterocycloalkyl,
A is a 3- to 12-membered heterocycle, and
R₅ and R₆ are each independently hydrogen, nitro, amino, halogen, hydroxy, cyano, C₁₋₆ alkyl, C₁₋₆ haloalkyl, C₂₋₆ alkenyl, C₂₋₆ alkynyl, C₁₋₆ alkoxy, amino-C₁₋₆ alkoxy, aminocarbonyl, C₁₋₆ alkylaminocarbonyl, diC₁₋₆ alkylaminocarbonyl, C₁₋₆ alkylcarbonylamino, C₁₋₆ alkylamino, C₁₋₆ alkylamino-C₁₋₆ alkoxy, C₆₋₁₀ aryl, C₆₋₁₀ aryl-C₁₋₄ alkyl, or 5- to 9-membered heteroaryl,
wherein R₅ and R₆ are each independently optionally substituted with C₁₋₆ alkyl; C₁₋₆ alkyl or C₁₋₆ alkylamino-C₁₋₆ alkyl, substituted with any one of C₁₋₆ alkoxy-C₁₋₆ alkylamino, 3- to 9-membered cycloalkyl, and 3- to 9-membered heterocycloalkyl; 3-to 9-membered cycloalkyl; or 3- to 9-membered heterocycloalkyl,
the cycloalkyl or the heterocycloalkyl optionally has one or more substituents selected from the group consisting of halogen, oxo, cyano, hydroxy, hydroxy-C₁₋₆ alkyl, amino, diC₁₋₆ alkylamino, C₁₋₆ alkyl, C₁₋₆ alkoxy, and C₁₋₆ alkoxy-C₁₋₆ alkyl, and
the heterocycle, the heteroaryl, and the heterocycloalkyl each independently contain one or more heteroatoms selected from the group consisting of N, O, and S.

In addition, the C₁₋₆ alkyl may include C₁₋₃ alkyl, C₃₋₆ alkyl, and the like. In addition, the C₁₋₆ alkoxy may include C₁₋₃ alkoxy, C₃₋₆ alkoxy, and the like.

In addition, the 3- to 12-membered heterocycle may include a 4- to 12-membered heterocycle, a 4- to 10-membered heterocycle, a 4- or 7-membered heterocycle, a 7- to 12-membered heterocycle, and the like. In addition, the heterocycle may include an aromatic ring or a non-aromatic ring. As an example, the 3- to 12-membered heterocycle may contain one or more aromatic rings. For example, the heterocycle may be quinoline, quinazoline, pyridine, pyrimidine, thienopyridine, pyrrolopyridine, pyrazolopyridine, imidazopyridine, pyrrolopyrimidine, dihydropyrrolopyrimidine, furopyridine, pyrazolopyrimidine, purine, indazole, azetidine, diazetidine, pyrrolidine, pyrrole, imidazolidine, imidazole, pyrazolidine, pyrazole, oxazolidine, oxazole, isoxazolidine, isoxazole, thiazolidine, thiazole, isothiazolidine, isothiazole, piperidine, pyridine, piperazine, diazine, morpholine, thiomorpholine, azepane, diazepane, or the like.

According to an embodiment, in Formula 2, R₁ to R₄ may each independently be hydrogen, C₁₋₄ haloalkyl, or halogen. Here, the halogen may be F, Cl, Br, or I. Specifically, R₁ may be hydrogen, trifluoromethyl, or fluoro; R₂ may be hydrogen; R₃ may be fluoro; and R₄ may be hydrogen.

According to another embodiment, in Formula 2, X may be O or -CH(-Rx)- and Rx may be hydrogen or C₁₋₆ alkyl.

According to yet another embodiment, in Formula 2, A may be quinoline or thienopyridine.

The thienopyridine may be represented by the following structural formula:

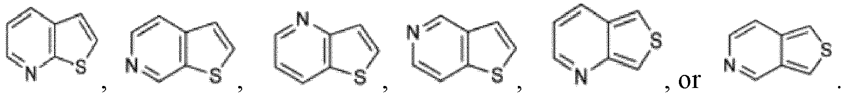

The pyrrolopyridine may be represented by the following structural formula:

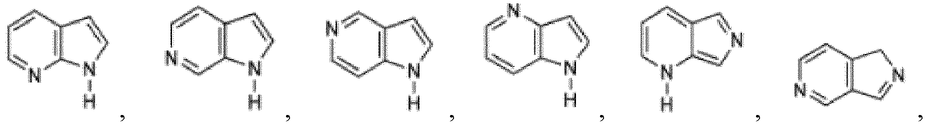

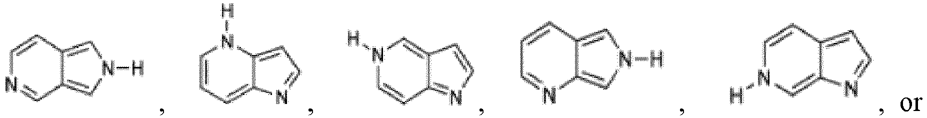

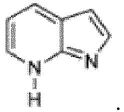

The pyrrolopyrimidine may be represented by the following structural formula:

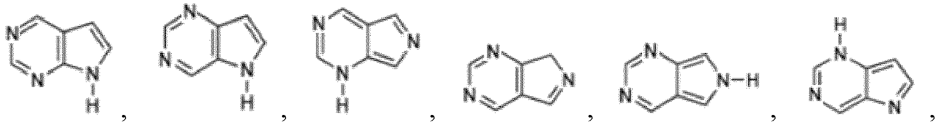

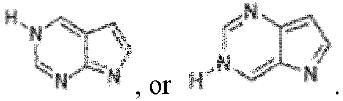

The dihydropyrrolopyrimidine may be represented by the following structural formula: 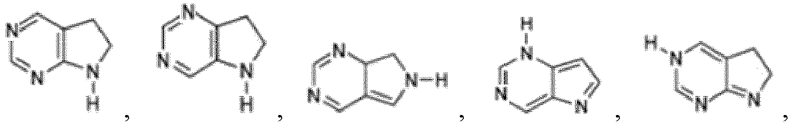 or

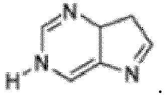

The pyrazolopyridine may be represented by the following structural formula:

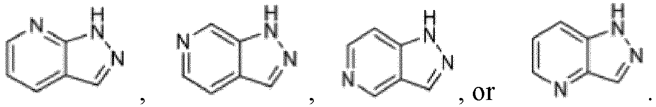

The pyrazolopyrimidine may be represented by the following structuralformula:

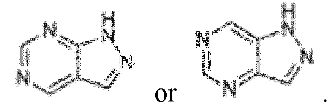

The imidazopyridine may be represented by the following structural formula:

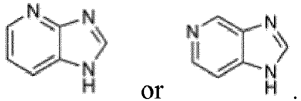

The furopyridine may be represented by the following structural formula:

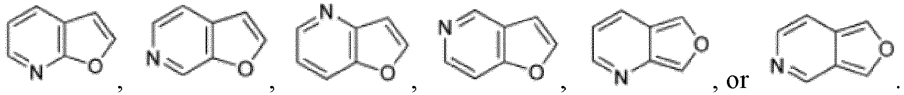

The purine may be represented by the following structural formula:

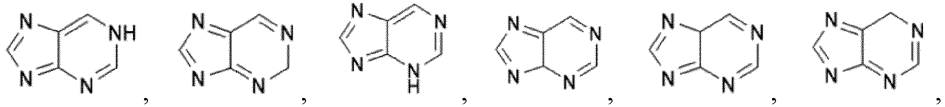

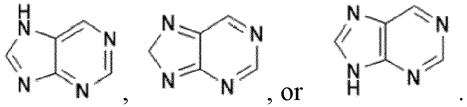

According to still yet another embodiment, in Formula 2, R₅ and R₆ are each independently hydrogen, amino, halogen, cyano, C₁₋₆ alkyl, C₁₋₆ haloalkyl, C₁₋₆ alkoxy, amino-C₁₋₆ alkoxy, aminocarbonyl, C₁₋₆ alkylaminocarbonyl, diC₁₋₆ alkylcarbonylamino, C₁₋₆ alkylcarbonylamino, C₁₋₆ alkylamino, C₁₋₆ alkylamino-C₁₋₆ alkoxy, or 5- to 9- membered heteroaryl, wherein R₅ and R₆ may each independently be optionally substituted with C₁₋₆ alkyl; C₁₋₆ alkyl or C₁₋₆ alkylamino-C₁₋₆ alkyl, substituted with any one of C₁₋₆ alkoxy-C₁₋₆ alkylamino, 3- to 9-membered cycloalkyl, and 3- to 9-membered heterocycloalkyl; 3- to 9-membered cycloalkyl; or 3- to 9-membered heterocycloalkyl; the cycloalkyl or the heterocycloalkyl may optionally have one or more substituents selected from the group consisting of halogen, oxo, cyano, hydroxy, hydroxy-C₁₋₆ alkyl, amino, diC₁₋₆ alkylamino, C₁₋₆ alkyl, C₁₋₆ alkoxy, and C₁₋₆ alkoxy-C₁₋₆ alkyl; and the heteroaryl and the heterocycloalkyl may each independently contain one or more heteroatoms selected from the group consisting of N, O, and S.

Specifically, the heteroaryl may be pyridinyl, imidazolyl, or pyrazolyl; the heterocycloalkyl may be azetidinyl, pyrrolidinyl, tetrahydropyranyl, morpholino, morpholinyl, dioxidothiomorpholino, piperazinyl, piperidinyl, or oxetanyl; and the cycloalkyl may be cyclobutyl, cyclopentyl, or cyclohexyl.

In addition, in a case where the heteroaryl or the heterocycloalkyl contains one or more N atoms, substitution may be made at any one of the N atomic positions. However, there is no particular limitation thereon.

According to still yet another embodiment, in Formula 2, R₁ and R₂ are hydrogen, C₁₋₄ haloalkyl, or halogen; R₃ and R₄ hydrogen or halogen; X is O or -CH(-Rx)- and Rx is hydrogen or C₁₋₄ alkyl; A is quinoline or thienopyridine; and R₅ and R₆ are each independently hydrogen, amino, halogen, cyano, C₁₋₆ alkyl, C₁₋₆ haloalkyl, C₁₋₆ alkoxy, amino-C₁₋₆ alkoxy, aminocarbonyl, C₁₋₆ alkylaminocarbonyl, diC₁₋₆ alkylcarbonylamino, C₁₋₆ alkylcarbonylamino, C₁₋₆ alkylamino, C₁₋₆ alkylamino-C₁₋₆ alkoxy, or 5- to 9-membered heteroaryl, wherein R₅ and R₆ may each independently be optionally substituted with C₁₋₆ alkyl; C₁₋₆ alkyl or C₁₋₆ alkylamino-C₁₋₆ alkyl, substituted with any one of C₁₋₆ alkoxy-C₁₋₆ alkylamino, 3- to 9-membered cycloalkyl, and 3- to 9-membered heterocycloalkyl; 3- to 9-membered cycloalkyl; or 3- to 9-membered heterocycloalkyl; the cycloalkyl or the heterocycloalkyl may optionally have one or more substituents selected from the group consisting of halogen, oxo, cyano, hydroxy, hydroxy-C₁₋₆ alkyl, amino, diC₁₋₆ alkylamino, C₁₋₆ alkyl, C₁₋₆ alkoxy, and C₁₋₆ alkoxy-C₁₋₆ alkyl; and the heteroaryl and the heterocycloalkyl may each independently contain one or more heteroatoms selected from the group consisting of N, O, and S.

According to still yet another embodiment, in Formula 2, R₅ and R₆ are each independently hydrogen, nitro, amino, halogen, hydroxy, cyano, C₁₋₆ alkyl, C₁₋₆ haloalkyl, C₁₋₆ alkoxy, amino-C₁₋₆ alkoxy, aminocarbonyl, C₁₋₆ alkylaminocarbonyl, diC₁₋₆ alkylaminocarbonyl, C₁₋₆ alkylcarbonylamino, C₁₋₆ alkylamino, C₁₋₆ alkylamino-C_{1- 6} alkoxy, C₆₋₁₀ aryl, C₆₋₁₀ aryl-C₁₋₄ alkyl, or 5- to 9-membered heteroaryl, wherein the amino, the alkyl, the alkoxy, the aryl, and the heteroaryl may each independently be optionally substituted with C₁₋₆ alkyl; C₁₋₆ alkyl or C₁₋₆ alkylamino-C₁₋₆ alkyl, substituted with any one of C₁₋₆ alkoxy-C₁₋₆ alkylamino, 3- to 9-membered cycloalkyl, and 3- to 9-membered heterocycloalkyl; 3- to 9-membered cycloalkyl; or 3- to 9-membered heterocycloalkyl; the cycloalkyl or the heterocycloalkyl may optionally have one or more substituents selected from the group consisting of halogen, oxo, cyano, hydroxy, hydroxy-C₁₋₆ alkyl, amino, diC₁₋₆ alkylamino, C₁₋₆ alkyl, C₁₋₆ alkoxy, and C₁₋₆ alkoxy-C₁₋₆ alkyl; and the heterocycle, the heteroaryl and the heterocycloalkyl may each independently contain one or more heteroatoms selected from the group consisting of N, O, and S.

According to still yet another embodiment, in Formula 2, R₅ and R₆ may not be, at the same time, C₆₋₁₀ aryl, C₆₋₁₀ aryl-C₁₋₄ alkyl, or 5- to 9-membered heteroaryl. According to still yet another embodiment, in Formula 2, R₅ and R₆ may not be substituted, at the same time, with C₁₋₆ alkyl or C₁₋₆ alkylamino-C₁₋₆ alkyl, substituted with any one of 3- to 9-membered cycloalkyl and 3- to 9-membered heterocycloalkyl; 3- to 9-membered cycloalkyl; or 3- to 9-membered heterocycloalkyl. As a specific example, in a case where R₅ contains a ring such as aryl or heteroaryl, R₆ may not contain this ring at the same time. In addition, in a case where R₅ is substituted with a group containing a ring such as cycloalkyl or heterocycloalkyl, R₆ may not be substituted, at the same time, with a group containing this ring.

As a more specific example, R₅ may be C₆₋₁₀ aryl, C₆₋₁₀ aryl-C₁₋₄ alkyl, or 5- to 9-membered heteroaryl; and R₆ may be hydrogen, nitro, amino, halogen, hydroxy, cyano, C₁₋₆ alkyl, C₁₋₆ haloalkyl, C₁₋₆ alkoxy, amino-C₁₋₆ alkoxy, aminocarbonyl, C₁₋₆ alkylaminocarbonyl, diC₁₋₆ alkylaminocarbonyl, C₁₋₆ alkylcarbonylamino, C₁₋₆ alkylamino, or C₁₋₆ alkylamino-C₁₋₆ alkoxy. Here, R₅ may be optionally substituted with C₁₋₆ alkyl; or C₁₋₆ alkyl or C₁₋₆ alkylamino-C₁₋₆ alkyl, substituted with any one of C₁₋₆ alkoxy-C₁₋₆ alkylamino, 3- to 9-membered cycloalkyl, and 3- to 9-membered heterocycloalkyl. In addition, R₆ may be optionally substituted with 3- to 9-membered cycloalkyl or 3- to 9-membered heterocycloalkyl. Here, the cycloalkyl or the heterocycloalkyl may optionally have one or more substituents selected from the group consisting of halogen, oxo, cyano, hydroxy, hydroxy-C₁₋₆ alkyl, amino, diC₁₋₆ alkylamino, C₁₋₆ alkyl, C₁₋₆ alkoxy, and C₁₋₆ alkoxy-C₁₋₆ alkyl; and the heteroaryl and the heterocycloalkyl may each independently contain one or more heteroatoms selected from the group consisting of N, O, and S.

According to still yet another embodiment, in Formula 1, R₅ and R₆ are each independently hydrogen, amino, halogen, hydroxy, C₁₋₆ alkoxy, aminocarbonyl, C₁₋₆ alkylaminocarbonyl, diC₁₋₆ alkylaminocarbonyl, C₁₋₆ alkylcarbonylamino, cyano, C₁₋₄ haloalkyl, C₁₋₆ alkyl, 5- or 9-membered heteroaryl, Ax-(CH₂)ₐ-L1-(CH₂)_{b}-L2-, or Ax-(CH₂)ₐ-L1-(CH₂)_{b}-L2-pyridinyl; Ax is C₃₋₆ cycloalkyl or 3-6 membered heterocycloalkyl; L1 and L2 are each independently a single bond, -O-, -NH-, -C(=O)-NH-, or -NH-C(=O)-; and a and b are each independently an integer of 0 to 3, provided that in a case where b is 0, L2 is a single bond, wherein the cycloalkyl, the heteroaryl, and the heterocycloalkyl may each independently optionally have one or two substituents selected from the group consisting of halogen, oxo, cyano, hydroxy, hydroxymethyl, C₁₋₆ alkyl, methoxy, methoxymethyl, dimethylamino, and methoxyethylaminomethyl; the heteroaryl and the heterocycloalkyl may each independently contain one or more heteroatoms selected from the group consisting of N, O, and S; and the heteroaryl and the heterocycloalkyl may each independently contain one or more heteroatoms selected from the group consisting of N, O, and S.

According to one embodiment, the compound of Formula 2 may be represented by Formula 2a below. 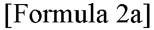 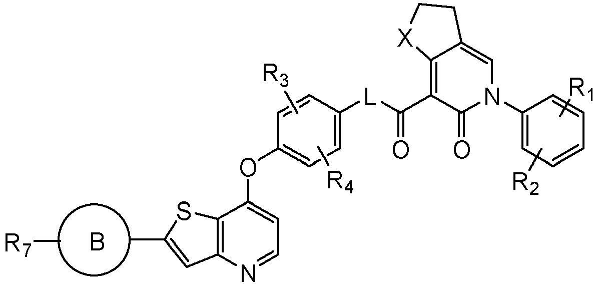 in the formula,
X, L, R₁, R₂, R₃, and R₄ are as defined in Formula 2,
B is a 5- or 6-membered aromatic heterocycle containing 1 or 2 N atoms, and
R₇ is C₁₋₆ alkyl; or C₁₋₆ alkyl or C₁₋₆ alkylamino-C₁₋₆ alkyl, substituted with any one of C₁₋₆ alkoxy-C₁₋₆ alkylamino, 3- to 9-membered cycloalkyl, and 3- to 9-membered heterocycloalkyl,
wherein the cycloalkyl or the heterocycloalkyl optionally has one or more substituents selected from the group consisting of halogen, oxo, cyano, hydroxy, hydroxy-C₁₋₆ alkyl, amino, diC₁₋₆ alkylamino, C₁₋₆ alkyl, C₁₋₆ alkoxy, and C₁₋₆ alkoxy-C₁₋₆ alkyl, and
the heterocycloalkyl contains 1 to 4 heteroatoms selected from the group consisting of N, O, and S.

Specific examples of the compound represented by Formula 2a are as follows:
b1) N-(3-fluoro-4-((2-(5-(((2-methoxyethyl)amino)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
b2) N-(3-fluoro-4-((2-(5-(((2-methoxyethyl)amino)methyl)pyridin-2-yl)thieno[3,2-b]pyridin-7-yl)oxy)phenyl)-6-oxo-5-phenyl-2,3,5,6-tetrahydrofuro[3,2-c]pyridine-7-carboxamide;
b3) N-(3-fluoro-4-((2-(pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
b4) N-(3-fluoro-4-((2-pyridin-2-yl)thieno[3,2-b]pyridin-7-yl)oxy)phenyl)-6-oxo-5-phenyl-2,3,5,6-tetrahydrofuro[3,2-c]pyridine-7-carboxamide;
b5) N-(3-fluoro-4-((2-(5-(morpholinomethyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
b6) N-(3-fluoro-4-((2-(5-(morpholinomethyl)pyridin-2-yl)thieno[3,2-b]pyridin-7-yl)oxy)phenyl)-6-oxo-5-phenyl-2,3,5,6-tetrahydrofuro[3,2-c]pyridine-7-carboxamide;
b7) N-(3-fluoro-4-((2-(5-morpholinomethyl)pyridin-2-yl)thieno[3,2-b]pyridin-7-yl)phenyl)-2-(4-fluorophenyl)-3-oxo-3,5,6,7-tetrahydro-2H-cyclopenta[c]pyridine-4-carboxamide;
b8) N-(3-fluoro-4-((2-(1-methyl-1H-imidazol-4-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
b9) N-(3-fluoro-4-((2-(1-methyl-1H-imidazol-4-yl)thieno[3,2-b]pyridin-7-yl)oxy)phenyl)-6-oxo-5-phenyl-2,3,5,6-tetrahydrofuro[3,2-c]pyridine-7-carboxamide;
b10) N-(3-fluoro-4-((2-(1-methyl-1H-pyrazol-4-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
b11) N-(3-fluoro-4-((2-(5-((3-hydroxyazetidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
b12) N-(3-fluoro-4-((2-(5-((3-(hydroxymethyl)azetidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
b13) N-(3-fluoro-4-((2-(5-((3-hydroxy-3-methylazetidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
b14) N-(3-fluoro-4-((2-(5-((3-methoxyazetidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
b15) N-(3-fluoro-4-((2-(5-((3-(methoxymethyl)azetidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
b16) N-(3-fluoro-4-((2-(5-((3-methoxy-3-methylazetidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
b 17) N-(3-fluoro-4-((2-(5-((3-fluoroazetidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
b18) N-(4-((2-(5-((3,3-difluoroazetidin-1-yl)methyl)pyridin-2-yl)thieno[3,2-b]pyridin-7-yl)oxy)-3-fluorophenyl)-5-(4-fluorophenyl)-6-oxo-2,3,5,6-tetrahydrofuro[3,2-c]pyridine-7-carboxamide;
b19) N-(4-((2-(5-((3-cyanoazetidin-1-yl)methyl)pyridin-2-yl)thieno[3,2-b]pyridin-7-yl)oxy)-3-fluorophenyl)-5-(4-fluorophenyl)-6-oxo-2,3,5,6-tetrahydrofuro[3,2-c]pyridine-7-carboxamide;
b20) N-(4-((2-(5-((3-(dimethylamino)azetidin-1-yl)methyl)pyridin-2-yl)thieno[3,2-b]pyridin-7-yl)oxy)-3-fluorophenyl)-5-(4-fluorophenyl)-6-oxo-2,3,5,6-tetrahydrofuro[3,2-c]pyridine-7-carboxamide;
b21) N-(3-fluoro-4-((2-(5-((3-methylazetidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
b22) N-(4-((2-(5-((3,3-dimethylazetidin-1-yl)methyl)pyridin-2-yl)thieno[3,2-b]pyridin-7-yl)oxy)-3-fluorophenyl)-5-(4-fluorophenyl)-6-oxo-2,3,5,6-tetrahydrofuro[3,2-c]pyridine-7-carboxamide;
b23) N-(3-fluoro-4-((2-(5-((3-hydroxypyrrolidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
b24) N-(3-fluoro-4-((2-(5-((3-hydroxy-3-methylpyrrolidin-1-yl)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
b25) N-(3-fluoro-4-((2-(5-((3-methoxypyrrolidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
b26) N-(3-fluoro-4-((2-(5-(pyrrolidin-1-ylmethyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
b27) N-(3-fluoro-4-((2-(5-((3-fluoropyrrolidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
b28) N-(4-((2-(5-((3,3-difluoropyrrolidin-1-yl)methyl)pyridin-2-yl)thieno[3,2-b]pyridine-7-yl)oxy)-3-fluorophenyl)-5-(4-fluorophenyl)-6-oxo-2,3,5,6-tetrahydrofuro[3,2-c]pyridine-7-carboxamide;
b29) N-(3-fluoro-4-((2-(5-((4-hydroxypiperidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
b30) N-(3-fluoro-4-((2-(5-((3-hydroxypiperidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
b31) N-(3-fluoro-4-((2-(5-((4-methoxypiperidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
b32) N-(3-fluoro-4-((2-(5-((3-methoxypiperidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
b33) N-(3-fluoro-4-((2-(5-(piperidin-1-ylmethyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
b34) N-(3-fluoro-4-((2-(5-((4-fluoropiperidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
b35) N-(4-((2-(5-((4,4-difluoropiperidin-1-yl)methyl)pyridin-2-yl)thieno[3,2-b]pyridine-7-yl)oxy)-3-fluorophenyl)-5-(4-fluorophenyl)-6-oxo-2,3,5,6-tetrahydrofuro[3,2-c]pyridine-7-carboxamide;
b36) N-(3-fluoro-4-((2-(5-((4-methylpiperidin-1-yl)methyl)pyridin-2-yl)thieno[3,2-b]pyridine-7-yl)oxy)phenyl)-5-(4-fluorophenyl)-6-oxo-2,3,5,6-tetrahydrofuro[3,2-c]pyridine-7-carboxamide;
b37) N-(4-((2-(5-((4,4-dimethylpiperidin-1-yl)methyl)pyridin-2-yl)thieno[3,2-b]pyridin-7-yl)oxy)-3-fluorophenyl)-5-(4-fluorophenyl)-6-oxo-2,3,5,6-tetrahydrofuro[3,2-c]pyridine-7-carboxamide;
b38) N-(3-fluoro-4-((2-(5-((((3-hydroxycyclohexyl)methyl)amino)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide; and
b39) N-(3-fluoro-4-((2-(5-(((tetrahydro-2H-pyran-4-yl)amino)methyl)pyridin-2-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide.

According to still yet another embodiment, the compound of Formula 2 may have a structure of Formula 2b: 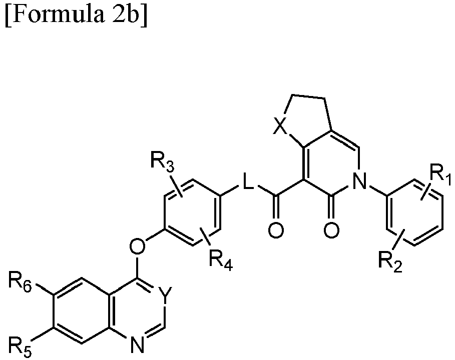 in the formula,
X, L, R₁, R₂, R₃, and R₄ are as defined in Formula 2,
Y is -N= or -CH=, and
R₅ and R₆ are each independently hydrogen, amino, halogen, cyano, C₁₋₆ alkyl, C₁₋₆ haloalkyl, C₁₋₆ alkoxy, amino-C₁₋₆ alkoxy, aminocarbonyl, C₁₋₆ alkylaminocarbonyl, diC₁₋₆ alkylcarbonylamino, C₁₋₆ alkylcarbonylamino, C₁₋₆ alkylamino, or C₁₋₆ alkylamino-C₁₋₆ alkoxy,
wherein R₅ and R₆ are each independently optionally substituted with 3- to 9-membered cycloalkyl or 3- to 9-membered heterocycloalkyl,
the cycloalkyl or the heterocycloalkyl optionally has one or more substituents selected from the group consisting of halogen, oxo, cyano, hydroxy, hydroxy-C₁₋₆ alkyl, amino, diC₁₋₆ alkylamino, C₁₋₆ alkyl, C₁₋₆ alkoxy, and C₁₋₆ alkoxy-C₁₋₆ alkyl, and
the heterocycloalkyl contains 1 to 4 heteroatoms selected from the group consisting of N, O, and S.

Specific examples of the compound represented by Formula 2b are as follows:
b40) N-(4-((6,7-dimethoxyquinolin-4-yl)oxy)-3-fluorophenyl)-5-(4-fluorophenyl)-6-oxo-2,3,5,6-tetrahydrofuro[3,2-c]pyridine-7-carboxamide;
b41) N-(4-((6,7-dimethoxyquinolin-4-yl)oxy)-3-fluorophenyl)-6-oxo-5-phenyl-2,3,5,6-tetrahydrofuro[3,2-c]pyridine-7-carboxamide;
b42) N-(4-((6,7-dimethoxyquinolin-4-yl)oxy)-3-fluorophenyl)-6-oxo-5-(4-(trifluoromethyl)phenyl)-2,3,5,6-tetrahydrofuro[3,2-c]pyridine-7-carboxamide;
b43) N-(4-((6,7-dimethoxyquinolin-4-yl)oxy)-3-fluorophenyl)-6-oxo-5-(3-fluorophenyl)-2,3,5,6-tetrahydrofuro[3,2-c]pyridine-7-carboxamide;
b44) N-(3-fluoro-4-((6-methoxyquinolin-4-yl)oxy)phenyl)-5-(4-fluorophenyl)-6-oxo-2,3,5,6-tetrahydrofuro[3,2-c]pyridine-7-carboxamide;
b45) N-(3-fluoro-4-((7-methoxyquinolin-4-yl)oxy)phenyl)-5-(4-fluorophenyl)-6-oxo-2,3,5,6-tetrahydrofuro[3,2-c]pyridine-7-carboxamide;
b46) N-(4-((6,7-dimethoxyquinazolin-4-yl)oxy)-3-fluorophenyl)-5-(4-fluorophenyl)-6-oxo-2,3,5,6-tetrahydrofuro[3,2-c]pyridine-7-carboxamide;
b47) N-(4-((6-carbamoyl-7-methoxyquinolin-4-yl)oxy)-3-fluorophenyl)-5-(4-fluorophenyl)-6-oxo-2,3,5,6-tetrahydrofuro[3,2-c]pyridine-7-carboxamide;
b48) N-(3-fluoro-4-((7-methoxy-6-(methylcarbamoyl)quinolin-4-yl)oxy)phenyl)-5-(4-fluorophenyl)-6-oxo-2,3,5,6-tetrahydrofuro[3,2-c]pyridine-7-carboxamide;
b49) N-(4-((6-(dimethylcarbamoyl)-7-methoxyquinolin-4-yl)oxy)-3-fluorophenyl)-5-(4-fluorophenyl)-6-oxo-2,3,5,6-tetrahydrofuro[3,2-c]pyridine-7-carboxamide;
b50) N-(3-fluoro-4-((7-methoxy-6-((2-morpholinoethyl)carbamoyl)quinolin-4-yl)oxy)phenyl)-5-(4-fluorophenyl)-6-oxo-2,3,5,6-tetrahydrofuro[3,2-c]pyridine-7-carboxamide;
b51) N-(4-((6-(ethylcarbamoyl)-7-methoxyquinolin-4-yl)oxy)-3-fluorophenyl)-5-(4-fluorophenyl)-6-oxo-2,3,5,6-tetrahydrofuro[3,2-c]pyridine-7-carboxamide;
b52) N-(4-((6-acetamido-7-methoxyquinolin-4-yl)oxy)-3-fluorophenyl)-5-(4-fluorophenyl)-6-oxo-2,3,5,6-tetrahydrofuro[3,2-c]pyridine-7-carboxamide;
b53) N-(3-fluoro-4-((7-methoxy-6-(2-morpholinoacetamido)quinolin-4-yl)oxy)phenyl)-5-(4-fluorophenyl)-6-oxo-2,3,5,6-tetrahydrofuro[3,2-c]pyridine-7-carboxamide;
b76) N-(3-fluoro-4-((6-methoxy-7-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
b77) N-(3-fluoro-4-((6-methoxy-7-(3-morpholinopropoxy)quinolin-4-yl)oxy)phenyl)-2-(4-fluorophenyl)-3-oxo-3,5,6,7-tetrahydro-2H-cyclopenta[c]pyridine-4-carboxamide;
b78) N-(3-fluoro-4-((6-methoxy-7-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
b79) N-(3-fluoro-4-((6-methoxy-7-(2-morpholinoethoxy)quinolin-4-yl)oxy)phenyl)-6-oxo-5-phenyl-2,3,5,6-tetrahydrofuro[3,2-c]pyridine-7-carboxamide;
b80) N-(3-fluoro-4-((6-methoxy-7-(3-(4-methylpiperazin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
b81) N-(3-fluoro-4-((7-(3-(3-hydroxyazetidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
b82) N-(3-fluoro-4-((7-(3-(3-hydroxy-3-methylazetidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
b83) N-(3-fluoro-4-((7-(3-(3-(hydroxymethyl)azetidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
b84) N-(3-fluoro-4-((6-methoxy-7-(3-(3-methoxyazetidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
b85) N-(3-fluoro-4-((6-methoxy-7-(3-(3-methoxy-3-methylazetidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
b86) N-(3-fluoro-4-((7-(3-(3-fluoroazetidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
b87) N-(4-((7-(3-(3,3-difluoroazetidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
b88) N-(4-((7-(3-(3-cyanoazetidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
b89) N-(3-fluoro-4-((6-methoxy-7-(3-(3-methylazetidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
b90) N-(3-fluoro-4-((7-(3-(3-hydroxypyrrolidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
b91) N-(3-fluoro-4-((7-(3-(3-hydroxy-3-methylpyrrolidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
b92) N-(3-fluoro-4-((6-methoxy-7-(3-(3-methoxypyrrolidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
b93) N-(3-fluoro-4-((6-methoxy-7-(3-(pyrrolidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
b94) N-(3-fluoro-4-((7-(3-(3-fluoropyrrolidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
b95) N-(4-((7-(3-(3,3-difluoropyrrolidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
b96) N-(3-fluoro-4-((7-(3-(4-hydroxypiperidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
b97) N-(3-fluoro-4-((7-(3-(3-hydroxypiperidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
b98) N-(3-fluoro-4-((7-(3-(4-hydroxy-4-methylpiperidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
b99) N-(3-fluoro-4-((6-methoxy-7-(3-(4-methoxypiperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
b100) N-(3-fluoro-4-((6-methoxy-7-(3-(3-methoxypiperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
b101) N-(3-fluoro-4-((6-methoxy-7-(3-(4-oxopiperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
b102) N-(4-((7-(3-(1,1-dioxidothiomorpholino)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
b103) N-(3-fluoro-4-((6-methoxy-7-(3-(piperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
b 104) N-(3-fluoro-4-((7-(3-(4-fluoropiperidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
b105) N-(4-((7-(3-(4,4-difluoropiperidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
b106) N-(3-fluoro-4-((6-methoxy-7-(3-(4-methylpiperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
b107) N-(4-((7-(3-(4,4-dimethylpiperidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
b108) N-(3-fluoro-4-((7-(3-((3-hydroxycyclobutyl)amino)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
b109) N-(3-fluoro-4-((6-methoxy-7-(3-((3-methoxycyclobutyl)amino)propoxy)quinolin-4-yl)oxy)phenyl)-5-(4-fluorophenyl)-6-oxo-2,3,5,6-tetrahydrofuro[3,2-c]pyridine-7-carboxamide;
b110) N-(3-fluoro-4-((6-methoxy-7-(3-(oxetan-3-ylamino)propoxy)quinolin-4-yl)oxy)phenyl)-5-(4-fluorophenyl)-6-oxo-2,3,5,6-tetrahydrofuro[3,2-c]pyridine-7-carboxamide;
b111) N-(3-fluoro-4-((6-methoxy-7-(3-((oxetan-3-ylmethyl)amino)propoxy)quinolin-4-yl)oxy)phenyl)-5-(4-fluorophenyl)-6-oxo-2,3,5,6-tetrahydrofuro[3,2-c]pyridine-7-carboxamide;
b112) N-(3-fluoro-4-((7-(3-((3-hydroxycyclopentyl)amino)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
b113) N-(3-fluoro-4-((7-(3-((3-hydroxycyclohexyl)amino)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
b114) N-(3-fluoro-4-((7-(3-(((3-hydroxycyclohexyl)methyl)amino)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
b 115) N-(3-fluoro-4-((6-methoxy-7-(3-((tetrahydro-2H-pyran-4-yl)amino)propoxy)quinolin-4-yl)oxy)phenyl)-5-(4-fluorophenyl)-6-oxo-2,3,5,6-tetrahydrofuro[3,2-c]pyridine-7-carboxamide;
b 116) N-(3-fluoro-4-((6-methoxy-7-(3-(((tetrahydro-2H-pyran-4-yl)methyl)amino)propoxy)quinolin-4-yl)oxy)phenyl)-5-(4-fluorophenyl)-6-oxo-2,3,5,6-tetrahydrofuro[3,2-c]pyridine-7-carboxamide;
b117) N-(3-fluoro-4-((7-(2-(3-hydroxyazetidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
b118) N-(3-fluoro-4-((7-(2-(3-hydroxy-3-methylazetidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
b119) N-(3-fluoro-4-((7-(2-(3-(hydroxymethyl)azetidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
b120) N-(3-fluoro-4-((6-methoxy-7-(2-(3-methoxyazetidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
b121) N-(3-fluoro-4-((6-methoxy-7-(2-(3-methoxy-3-methylazetidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
b122) N-(3-fluoro-4-((6-methoxy-7-(2-(3-(methoxymethyl)azetidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
b123) N-(3-fluoro-4-((7-(2-(3-fluoroazetidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
b124) N-(4-((7-(2-(3,3-difluoroazetidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
b125) N-(4-((7-(2-(3-ethynylazetidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
b126) N-(3-fluoro-4-((6-methoxy-7-(2-(3-methylazetidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
b127) N-(4-((7-(2-(3,3-dimethylazetidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
b128) N-(4-((7-(2-(3-(dimethylamino)azetidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
b129) N-(3-fluoro-4-((7-(2-(3-hydroxypyrrolidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
b130) N-(3-fluoro-4-((7-(2-(3-hydroxy-3-methylpyrrolidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
b131) N-(3-fluoro-4-((6-methoxy-7-(2-(3-methoxypyrrolidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
b132) N-(3-fluoro-4-((6-methoxy-7-(2-(pyrrolidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
b133) N-(3-fluoro-4-((7-(2-(3-fluoropyrrolidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
b134) N-(4-((7-(2-(3,3-difluoropyrrolidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
b135) N-(3-fluoro-4-((7-(2-(4-hydroxypiperidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
b136) N-(3-fluoro-4-((7-(2-(3-hydroxypiperidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
b137) N-(3-fluoro-4-((7-(2-(4-hydroxy-4-methylpiperidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
b138) N-(3 -fluoro-4-((6-methoxy-7-(2-(4-methoxypiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
b139) N-(3-fluoro-4-((6-methoxy-7-(2-(3-methoxypiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
b140) N-(3-fluoro-4-((6-methoxy-7-(2-(4-oxopiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
b141) N-(3-fluoro-4-((6-methoxy-7-(2-(3-oxopiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
b142) N-(4-((7-(2-(1,1-dioxidothiomorpholino)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
b143) N-(3-fluoro-4-((6-methoxy-7-(2-(piperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
b144) N-(3-fluoro-4-((7-(2-(4-fluoropiperidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
b145) N-(4-((7-(2-(4,4-difluoropiperidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
b146) N-(3-fluoro-4-((6-methoxy-7-(2-(4-methylpiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
b147) N-(4-((7-(2-(4,4-dimethylpiperidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
b148) N-(3-fluoro-4-((7-(2-((3-hydroxycyclobutyl)amino)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
b149) N-(3-fluoro-4-((6-methoxy-7-(2-((3-methoxycyclobutyl)amino)ethoxy)quinolin-4-yl)oxy)phenyl)-5-(4-fluorophenyl)-6-oxo-2,3,5,6-tetrahydrofuro[3,2-c]pyridine-7-carboxamide;
b150) N-(3-fluoro-4-((6-methoxy-7-(2-(oxetan-3-ylamino)ethoxy)quinolin-4-yl)oxy)phenyl)-5-(4-fluorophenyl)-6-oxo-2,3,5,6-tetrahydrofuro[3,2-c]pyridine-7-carboxamide;
b151) N-(3-fluoro-4-((6-methoxy-7-(2-((oxetan-3-ylmethyl)amino)ethoxy)quinolin-4-yl)oxy)phenyl)-5-(4-fluorophenyl)-6-oxo-2,3,5,6-tetrahydrofuro[3,2-c]pyridine-7-carboxamide;
b152) N-(3-fluoro-4-((7-(2-((4-hydroxycyclohexyl)amino)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
b153) N-(3-fluoro-4-((7-(2-((3-hydroxycyclohexyl)amino)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
b154) N-(3-fluoro-4-((7-(2-(((3-hydroxycyclohexyl)methyl)amino)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
b155) N-(3-fluoro-4-((6-methoxy-7-(2-((tetrahydro-2H-pyran-4-yl)amino)ethoxy)quinolin-4-yl)oxy)phenyl)-5-(4-fluorophenyl)-6-oxo-2,3,5,6-tetrahydrofuro[3,2-c]pyridine-7-carboxamide;
b156) N-(3-fluoro-4-((6-methoxy-7-(2-((4-methoxycyclohexyl)amino)ethoxy)quinolin-4-yl)oxy)phenyl)-5-(4-fluorophenyl)-6-oxo-2,3,5,6-tetrahydrofuro[3,2-c]pyridine-7-carboxamide;
b157) N-(3-fluoro-4-((7-(2-morpholinylethoxy)quinolin-4-yl)oxy)phenyl)-5-(4-fluorophenyl)-6-oxo-2,3,5,6-tetrahydrofuro[3,2-c]pyridine-7-carboxamide;
b158) N-(3-fluoro-4-((7-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
b159) N-(3-fluoro-4-((7-(3-morpholinopropoxy)quinolin-4-yl)oxy)phenyl)-2-(4-fluorophenyl)-3-oxo-3,5,6,7-tetrahydro-2H-cyclopenta[c]pyridine-4-carboxamide;
b160) N-(3-fluoro-4-((7-(3-(4-methylpiperazin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
b161) 7-(2-(3-fluoro-4-((7-(3-(piperidin-1-yl)propoxy)quinolin-4-yl)oxy)phenyl)acetyl)-5-(4-fluorophenyl)-3,5-dihydrofuro[3,2-c]pyridin-6(2H)-one;
b162) 7-(2-(3-fluoro-4-((7-(3-(4-methylpiperidin-1-yl)propoxy)quinolin-4-yl)oxy)phenyl)acetyl)-5-(4-fluorophenyl)-3,5-dihydrofuro[3,2-c]pyridin-6(2H)-one;
b163) N-(3-fluoro-4-((6-(methylcarbamoyl)-7-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
b164) N-(3-fluoro-4-((6-(methylcarbamoyl)-7-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
b165) N-(3-fluoro-4-((6-methoxy-7-(methylcarbamoyl)quinolin-4-yl)oxy)phenyl)-5-(4-fluorophenyl)-6-oxo-2,3,5,6-tetrahydrofuro[3,2-c]pyridine-7-carboxamide;
b166) N-(3-fluoro-4-((7-(methylcarbamoyl)-6-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
b167) N-(3-fluoro-4-((6-fluoro-7-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
b168) N-(3-fluoro-4-((6-fluoro-7-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
b169) N-(3-fluoro-4-((7-(2-morpholinoethoxy)-6-(trifluoromethyl)quinolin-4-yl)oxy)phenyl)-5-(4-fluorophenyl)-6-oxo-2,3,5,6-tetrahydrofuro[3,2-c]pyridine-7-carboxamide;
b170) N-(3-fluoro-4-((7-(3-morpholinopropoxy)-6-(trifluoromethyl)quinolin-4-yl)oxy)phenyl)-5-(4-fluorophenyl)-6-oxo-2,3,5,6-tetrahydrofuro[3,2-c]pyridine-7-carboxamide;
b171) N-(4-((6-chloro-7-(2-morpholinoethoxy)quinolin-4-yl)oxy)-3-fluorophenyl)-5-(4-fluorophenyl)-6-oxo-2,3,5,6-tetrahydrofuro[3,2-c]pyridine-7-carboxamide;
b172) N-(4-((6-chloro-7-(3-morpholinopropoxy)quinolin-4-yl)oxy)-3-fluorophenyl)-5-(4-fluorophenyl)-6-oxo-2,3,5,6-tetrahydrofuro[3,2-c]pyridine-7-carboxamide;
b173) N-(4-((6-cyano-7-(2-morpholinoethoxy)quinolin-4-yl)oxy)-3-fluorophenyl)-5-(4-fluorophenyl)-6-oxo-2,3,5,6-tetrahydrofuro[3,2-c]pyridine-7-carboxamide;
b174) N-(4-((6-cyano-7-(3-morpholinopropoxy)quinolin-4-yl)oxy)-3-fluorophenyl)-5-(4-fluorophenyl)-6-oxo-2,3,5,6-tetrahydrofuro[3,2-c]pyridine-7-carboxamide;
b175) N-(3-fluoro-4-((7-methoxy-6-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
b176) N-(3-fluoro-4-((7-methoxy-6-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
b177) N-(3-fluoro-4-((6-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
b178) N-(3-fluoro-4-((6-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
b179) N-(4-((6-amino-7-methoxyquinolin-4-yl)oxy)-3-fluorophenyl)-5-(4-fluorophenyl)-6-oxo-2,3,5,6-tetrahydrofuro[3,2-c]pyridine-7-carboxamide;
b180) N-(3-fluoro-4-((7-methoxy-6-(3-morpholinopropanamido)quinolin-4-yl)oxy)phenyl)-5-(4-fluorophenyl)-6-oxo-2,3,5,6-tetrahydrofuro[3,2-c]pyridine-7-carboxamide;
b181) N-(4-((7-amino-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
b182) N-(3-fluoro-4-((6-methoxy-7-(2-morpholinoacetamido)quinolin-4-yl)oxy)phenyl)-5-(4-fluorophenyl)-6-oxo-2,3,5,6-tetrahydrofuro[3,2-c]pyridine-7-carboxamide;
b183) N-(3-fluoro-4-((6-methoxy-7-(3-morpholinopropanamido)quinolin-4-yl)oxy)phenyl)-5-(4-fluorophenyl)-6-oxo-2,3,5,6-tetrahydrofuro[3,2-c]pyridine-7-carboxamide;
b184) N-(4-((7-acetamido-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
b185) N-(3-fluoro-4-((7-methoxy-6-((2-morpholinoethyl)amino)quinolin-4-yl)oxy)phenyl)-5-(4-fluorophenyl)-6-oxo-2,3,5,6-tetrahydrofuro[3,2-c]pyridine-7-carboxamide;
b186) N-(3-fluoro-4-((7-methoxy-6-((3-morpholinopropyl)amino)quinolin-4-yl)oxy)phenyl)-5-(4-fluorophenyl)-6-oxo-2,3,5,6-tetrahydrofuro[3,2-c]pyridine-7-carboxamide;
b187) N-(3-fluoro-4-((6-methoxy-7-((2-morpholinoethyl)amino)quinolin-4-yl)oxy)phenyl)-5-(4-fluorophenyl)-6-oxo-2,3,5,6-tetrahydrofuro[3,2-c]pyridine-7-carboxamide; and
b188) N-(3-fluoro-4-((6-methoxy-7-((3-morpholinopropyl)amino)quinolin-4-yl)oxy)phenyl)-5-(4-fluorophenyl)-6-oxo-2,3,5,6-tetrahydrofuro[3,2-c]pyridine-7-carboxamide.

Preferably, the compounds of Formula 2 may be selected from the group consisting of:
N-(3-fluoro-4-((2-(1-methyl-1H-imidazol-4-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
N-(3-fluoro-4-((6-methoxy-7-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide; and
N-(4-((7-(3-(3-cyanoazetidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide.

The compound of Formula 2 above used in the composition according to the present invention may be prepared by a method disclosed in Korean Patent Laid-open Publication No. 2021-0015730, and by other known methods and/or various methods based on the technology in the field of organic synthesis. Based on the above methods, various derivatives may be synthesized using an appropriate synthesis method according to the type of substituent.

According to a further embodiment, the compound comprised as an active ingredient in the pharmaceutical composition of the present invention may be represented by Formula 3 below. That is, the pharmaceutical composition according to the present invention may comprise the compound represented by Formula 3 below and a pharmaceutically acceptable salt thereof.

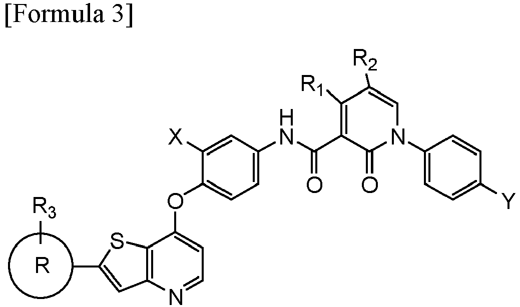

In Formula above,
X is hydrogen or halogen;
Y is hydrogen or halogen;
R₁ and R₂ are each independently hydrogen or C₁₋₁₀ alkoxy, or are connected to each other to form 5- to 10-membered heterocycloalkyl having heteroatom of O;
R is 5- to 7-membered heteroaryl having 1 or 2 heteroatoms of N;
R₃ is hydrogen or -(CH₂)ₙ-R₄;
wherein n is an integer of 0 to 10; and
R₄ is 5- to 10-membered heterocycle having 1 or 2 heteroatoms selected from the group consisting of N, O, and S.

According to an embodiment, in Formula 3 above, X and Y may each be hydrogen or fluoro.

According to another embodiment, in Formula 3 above, R₁ and R₂ may each independently be hydrogen, methyl, methoxy, or ethoxy and may not be hydrogens at the same time, or may be connected to each other to form 5- to 10-membered heterocycloalkyl having heteroatom of O.

According to another embodiment, in Formula 3 above, R may be 5- to 6-membered heteroaryl having 1 or 2 heteroatoms of N.

According to another embodiment, in Formula 3 above, R₃ is hydrogen or-(CH₂)ₙ-R₄, wherein n may be an integer of 0 to 4, and R₄ may be 6-membered heterocycle having heteroatoms of N and O.

According to another embodiment, in Formula 3 above, X and Y may be fluoro; R₁ and R₂ may each independently be ethoxy or hydrogen and may not be hydrogens at the same time, or may be connected to each other to form 5-membered heterocycloalkyl having heteroatom of O; R may be pyridine or imidazole; R₃ may be hydrogen or -(CH₂)-R₄; and R₄ may be 6-membered heterocycle having heteroatoms of N and O.

The compound of Formula 3 above may be prepared by a method disclosed in Korean Patent Laid-open Publication No. 2019-0106802 or 2021-0015730, and by other known methods and/or various methods based on the technology in the field of organic synthesis. Based on the above methods, various derivatives may be synthesized using an appropriate synthesis method according to the type of substituent.

As used herein, the term "halogen" refers to F, Cl, Br, or I unless otherwise stated.

The term "alkyl", unless otherwise specified, refers to a linear or branched saturated hydrocarbon moiety. For example, "C₁₋₁₀ alkyl" refers to an alkyl group having a skeleton of 1 to 10 carbon atoms. Specifically, the C₁₋₁₀ alkyl may include methyl, ethyl, n-propyl, i-propyl, n-butyl, i-butyl, t-butyl, n-pentyl, i-pentyl, t-pentyl, sec-pentyl, neopentyl, hexyl, heptyl, octyl, nonyl, decyl, and the like.

The term "haloalkyl" refers to an alkyl group substituted with one or more halogen atoms. Specifically, the haloalkyl may be an alkyl group substituted with two or more halogen atoms, the halogen atoms being the same or different.

The term "alkoxy", unless otherwise specified, refers to a group having the formula -O-alkyl, in which the alkyl is an alkyl group as defined above and is attached to a parent compound through an oxygen atom. The alkyl portion of the alkoxy group may have 1 to 20 carbon atoms (that is, C₁-C₂₀ alkoxy), 1 to 12 carbon atoms (that is, C₁-C₁₂ alkoxy), or 1 to 6 carbon atoms (that is, C₁-C₆ alkoxy). Examples of suitable alkoxy groups include methoxy (-O-CH₃ or -OMe), ethoxy (-OCH₂CH₃ or -OEt), t-butoxy (-OC(CH₃)₃ or -O-tBu), and the like.

The term "aryl" refers to an aromatic hydrocarbon radical derived by the removal of one hydrogen atom from a single carbon atom of a parent aromatic ring system. For example, the aryl group may have 6 to 20 carbon atoms, 6 to 14 carbon atoms, or 6 to 10 carbon atoms.

The term "cycloalkyl" refers to a saturated monocycle or polycycle that contains only carbon atoms in the ring. The cycloalkyl may have 3 to 7 carbon atoms as a monocycle, 7 to 12 carbon atoms as a bicycle, and up to about 20 carbon atoms as a polycycle.

The term "heterocycle" refers to an aromatic or non-aromatic ring having one or more heteroatoms, which may be saturated or unsaturated and may be monocyclic or polycyclic. For example, "4- to 10-membered heterocycle" refers to a heterocycle having a skeleton of a total of 4 to 10 atoms including at least one heteroatom and carbon atoms. Specifically, examples of the 4- to 10-membered heterocycle may include azetidine, diazetidine, pyrrolidine, pyrrole, imidazolidine, imidazole, pyrazolidine, pyrazole, oxazolidine, oxazole, isoxazolidine, isoxazole, thiazolidine, thiazole, isothiazolidine, isothiazole, piperidine, pyridine, piperazine, diazine, morpholine, thiomorpholine, azepane, diazepane, and the like. In the present specification, when "heterocycle" is described as a substituent, it may be used as a term encompassing "heteroaryl" and "heterocycloalkyl".

The term "heteroaryl" refers to an aromatic heterocyclyl having one or more heteroatoms in the ring. Non-limiting examples of the heteroaryl include pyridinyl, pyrrolyl, oxazolyl, indolyl, isoindolyl, furinyl, furanyl, thienyl, benzofuranyl, benzothiophenyl, carbazolyl, imidazolyl, thiazolyl, isoxazolyl, pyrazolyl, isothiazolyl, quinolyl, isoquinolyl, pyridazyl, pyrimidyl, pyrazyl, and the like (each of which may have one or more substituents on the ring).

The term "heterocycloalkyl" refers to a non-aromatic heterocyclyl having one or more heteroatoms in the ring. The heterocycloalkyl may have one or more carbon-carbon double bonds or carbon-heteroatom double bonds in the ring as long as the ring is not rendered aromatic by their presence. Non-limiting examples of the heterocycloalkyl include azetidinyl, aziridinyl, pyrrolidinyl, piperidinyl, piperazinyl, homopiperazinyl, morpholino, thiomorpholino, tetrahydrofuranyl, tetrahydrothiofuranyl, tetrahydropyranyl, pyranyl, and the like (each of which may have one or more substituents on the ring).

The term "heteroatom" refers to an atom other than carbon (C), and may specifically be a nitrogen (N), oxygen (O), or sulfur (S) atom. The above-mentioned heteroaryl and heterocycloalkyl contain one or more heteroatoms and may, for example, contain 1, 1 to 2,1 to 3, or 1 to 4 heteroatoms.

The term "substitution" refers to replacement of a hydrogen atom in a molecular structure with a substituent such that a chemically stable compound results from the substitution while not exceeding valence on the designated atom. For example, "group A is substituted with substituent B" or "group A has substituent B" means that a hydrogen atom bonded to an atom, such as carbon, which constitutes a skeleton of group A, is replaced with substituent B so that the group A and the substituent B form a covalent bond. Thus, it is substantially difficult or impossible for a group having no removable hydrogen atom to have a substituent. From this viewpoint, in a case where a range of combinations of various groups, which include groups that hardly have a substituent, with substituents are exemplified in the present specification, it should be interpreted that combinations of the groups, for which it is obvious that no substitution is possible, with the substituents are excluded from the range.

The pharmaceutical composition of the present invention may comprise, as an active ingredient, the compound represented by Formula 1 or Formula 2 or a pharmaceutically acceptable salt thereof in an amount of about 0.1% to about 90% by weight, specifically about 0.5% to about 75% by weight, and more specifically about 1% to about 50% by weight, with respect to a total weight of the composition.

The pharmaceutical composition of the present invention may comprise conventional and non-toxic pharmaceutically acceptable additives which are blended into a preparation according to a conventional method. For example, the pharmaceutical composition may further comprise a pharmaceutically acceptable carrier, diluent, or excipient.

Examples of the additives used in the composition of the present invention may include sweeteners, binders, solvents, dissolution aids, wetting agents, emulsifiers, isotonic agents, absorbents, disintegrants, antioxidants, preservatives, lubricants, fillers, flavoring agents, and the like. For example, the additives may include lactose, dextrose, sucrose, mannitol, sorbitol, cellulose, glycine, silica, talc, stearic acid, stearin, magnesium stearate, magnesium aluminosilicate, starch, gelatin, gum tragacanth, alginic acid, sodium alginate, methylcellulose, sodium carboxymethylcellulose, agar, water, ethanol, polyethylene glycol, polyvinylpyrrolidone, sodium chloride, calcium chloride, orange essence, strawberry essence, vanilla flavor, and the like.

The composition of the present invention may be made into various preparation forms for oral administration (for example, tablets, pills, powders, capsules, syrups, or emulsions) or parenteral administration (for example, intramuscular, intravenous, or subcutaneous injection).

Preferably, the composition of the present invention may be made into a preparation for oral administration, in which additives used may include cellulose, calcium silicate, corn starch, lactose, sucrose, dextrose, calcium phosphate, stearic acid, magnesium stearate, calcium stearate, gelatin, talc, surfactants, suspending agents, emulsifying agents, diluents, and the like.

Specifically, solid preparations for oral administration include tablets, pills, powders, granules, capsules, and the like, and such solid preparations can be formulated by mixing the composition with at least one excipient, such as starch, calcium carbonate, sucrose, lactose, and gelatin. In addition, in addition to simple excipients, a lubricant such as magnesium stearate and talc may be used.

In addition, as liquid preparations for oral administration, suspensions, emulsions, syrups, and the like may be exemplified, in which in addition to water and liquid paraffin which are commonly used simple diluents, various excipients, such as wetting agents, sweeteners, fragrances, and preservatives, may be included therein.

In addition, preparations for parenteral administration include sterilized aqueous solutions, non-aqueous solutions, suspensions, emulsions, lyophilized preparations, and suppositories. As the non-aqueous solution or the suspension, propylene glycol, polyethylene glycol, vegetable oil such as olive oil, injectable ester such as ethyl oleate, or the like may be used. As a base for the suppository, Witepsol, Macrogol, and Tween 61, cocoa butter, laurin fat, glycerogelatin, or the like may be used. On the other hand, the injection may comprise conventional additives such as solubilizing agents, isotonic agents, suspending agents, emulsifying agents, stabilizing agents, and preservatives.

The compound or composition of the present invention may be administered to a patient in a therapeutically effective amount or a pharmaceutically effective amount.

Here, the "therapeutically effective amount" or "pharmaceutically effective amount" refers to an amount of a compound or composition effective to prevent or treat a target disease, the amount being sufficient to treat the disease at a reasonable benefit/risk ratio, which is applicable to medical treatment, without causing adverse effects. A level of the effective amount may be determined depending on factors, including the patient's health status, disease type, disease severity, drug activity, drug sensitivity, method of administration, time of administration, route of administration and rate of excretion, duration of treatment, and drugs used simultaneously or in combination, and other factors well known in the medical field.

The compound or composition of the present invention may be administered as an individual therapeutic agent or administered in combination with other therapeutic agents, may be administered sequentially or simultaneously with a conventional therapeutic agent, and may be administered once or multiple times. It is important to administer an amount such that a maximum effect can be obtained with a minimum amount without adverse effects by taking all of the above-described factors into consideration, and such an amount can be readily determined by those skilled in the art.

Specifically, an effective amount of the compound contained in the composition of the present invention may vary depending on the patient's age, sex, and body weight. Generally, the compound may be administered in an amount of about 0.1 mg to about 1,000 mg, or about 5 mg to about 200 mg, per kg body weight, on a daily or every-other-day basis, or once or three times a day. However, the effective amount may increase or decrease depending on route of administration, disease severity, the patient's sex, body weight, and age, and the like. Thus, the scope of the present invention is not limited thereto.

Preferably, the compound or composition of the present invention may be administered for tumor therapy, in combination with chemotherapy, radiation therapy, immunotherapy, hormone therapy, bone marrow transplantation, stem cell replacement therapy, other biological therapies, surgical intervention, or a combination thereof. For example, the compound or composition of the present invention may be used as adjuvant therapy in combination with other long-term treatment strategies, or may be used to maintain the patient's condition after tumor regression is induced or chemoprophylactic therapy is applied in severely ill patients.

The pharmaceutical composition of the present invention may additionally comprise one or more active ingredients, and the additional active ingredient may be, but is not limited to, an anti-proliferative compound such as an aromatase inhibitor, anti-estrogen, a topoisomerase I inhibitor, a topoisomerase II inhibitor, a microtubule active compound, an alkylated compound, a histone deacetylase inhibitor, a compound that induces cell differentiation processes, a cyclooxygenase inhibitor, an MMP inhibitors, an mTOR inhibitor, an anti-neoplastic, anti-metabolite, a platinum compound, a compound that targets or decreases protein activity or lipid kinase activity, an anti-angiogenic compound, a compound that targets, decreases, or inhibits protein activity or lipid phosphatase activity, a gonadorelin agonist, anti-androgen, a methionine aminopeptidase inhibitor, bisphosphonate, a biological response modifier, an anti-proliferative antibody, a heparanase inhibitor, an inhibitor of Ras oncogenic isoforms, a telomerase inhibitor, a proteasome inhibitor, a compound used for treatment of hematologic malignancies, a compound that targets, decreases, or inhibits Flt-3 activity, an HSP90 inhibitor, a kinesin spindle protein inhibitor, a MEK inhibitor, leucovorin, an EDG binder, an anti-leukemic compound, a ribonucleotide reductase inhibitor, an S-adenosylmethionine decarboxylase inhibitor, a hemostatic steroid, a corticosteroid, another chemotherapy compound, or a photosensitizing compound.

The additional active ingredient may be a known anticancer agent. Non-limiting examples of the anticancer agent include DNA alkylating agents, such as mechlorethamine, chlorambucil, phenylalanine, mustard, cyclophosphamide, ifosfamide, carmustine (BCNU), lomustine (CCNU), streptozotocin, busulfan, thiotepa, cisplatin, and carboplatin; anti-cancer antibiotics, such as dactinomycin (actinomycin D), doxorubicin (adriamycin), daunorubicin, idarubicin, mitoxantrone, plicamycin, mitomycin C, and bleomycin; and plant alkaloids, such as vincristine, vinblastine, paclitaxel, docetaxel, etoposide, teniposide, topotecan, and irinotecan; and the like.

### Mode for Carrying out the Invention

Hereinafter, the present invention will be described in more detail by way of the following examples. However, the following examples are for illustrative purposes only, and the scope of the present invention is not limited thereto.

### Example 1: 4-ethoxy-N-[3-fluoro-4-({2-[5-(morpholinomethyl)pyridine-2-yl]thieno[3,2-b]pyridine-7-yl}oxy)phenyl]-1-(4-fluorophenyl)-2-oxo-1,2-dihydropyridine-3-carboxamide hydrochloride

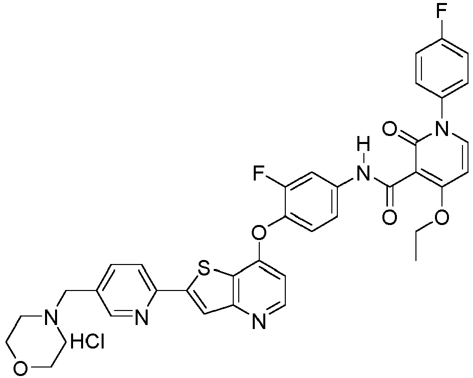

The title compound was synthesized by a method described in Example 31 of Korean Patent Laid-open Publication No. 2019-0106802.

¹H NMR (500MHz, DMSO-d₆) δ 10.66 (s, 1H), 8.81 (s, 1H), 8.60 (d, J = 5.0 Hz, 1H), 8.48 (s, 1H), 8.43 (d, J = 5.0 Hz, 1H), 8.23 (d, J = 5.0 Hz, 1H), 7.96 (d, J = 15.0 Hz, 1H), 7.87 (d, J = 5.0 Hz, 1H), 7.52-7.45 (m, 4H), 7.39-7.36 (m, 2H), 6.83 (d, J = 5.0 Hz, 1H), 6.53 (d, J = 10.0 Hz, 1H), 4.44 (s, 2H), 4.26 (qt, J = 5.0 H, 2H), 3.96-3.94 (m, 2H), 3.77 (t, J = 10.0 Hz, 2H), 3.32-3.30 (m, 2H), 3.14 (qt, J = 10.0 2H), 1.31 (t, J = 5.0 Hz, 3H)

### Example 2: 4-ethoxy-N-(3-fluoro-4-{[2-(5-{[(2-methoxyethyl)amino]methyl}pyridine-2-yl)thieno[3,2-b]pyridine-7-yl]oxy}phenyl)-2-oxo-1-phenyl-1,2-dihydropyridine-3-carboxamide hydrochloride

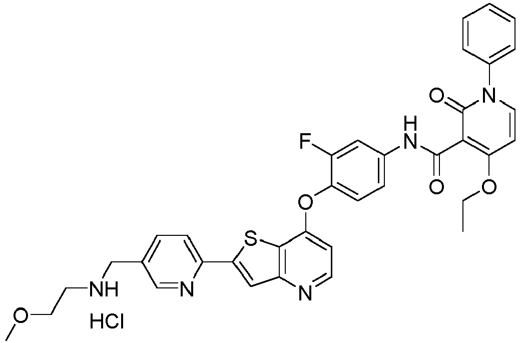

The title compound was synthesized by a method described in Example 1 of Korean Patent Laid-open Publication No. 2019-0106802.

¹H NMR (500 MHz, DMSO-d₆) δ 10.70 (brs, 1H), 9.50 (brs, 2H), 8.80 (s, 1H), 8.69 (d, J = 5.0 Hz, 1H), 8.47 (s, 1H), 8.44 (d, J = 5.0 Hz, 1H), 8.22 (dd, J = 10.0 and 5.0 Hz, 1H), 7.99 (d, J = 10.0 Hz, 1H), 7.88 (d, J = 5.0 Hz, 1H), 7.57-7.40 (m, 7H), 6.97 (d, J = 5.0 Hz, 1H), 6.53 (d, J = 5.0 Hz, 1H), 4.29-4.25 (m, 4H), 3.65 (t, J = 5.0 Hz, 2H), 3.31 (s, 3H), 3.16-3.12 (m, 2H), 1.31 (t, J = 5.0 Hz, 3H)

### Example 3: 4-ethoxy-N-{3-fluoro-4-[(2-{5-[(4-methylpiperazin-1-yl)methyl]pyridin-2-yl}thieno[3,2-b]pyridin-7-yl)oxy]phenyl}-2-oxo-1-phenyl-1,2-dihydropyridine-3-carboxamide hydrochloride

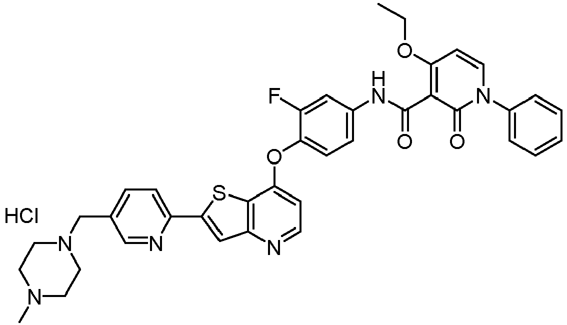

The title compound was synthesized by a method described in Example 34 of Korean Patent Laid-open Publication No. 2019-0106802.

¹H NMR (500MHz, DMSO-d₆) δ 10.68 (s, 1H), 8.82 (brs, 1H), 8.61 (d, J = 5.0 Hz, 1H), 8.47 (s, 1H), 8.41 (d, J = 5.0 Hz, 1H), 8.23 (s, 1H), 7.97 (d, J = 15.0 Hz, 1H), 7.87 (d, J = 5.0 Hz, 1H), 7.56-7.46 (m, 5H), 7.42-7.40 (m, 2H), 6.86 (d, J = 5.0 Hz, 1H), 6.52 (d, J = 5.0 Hz, 1H), 4.26 (qt, J = 5.0 H, 2H), 3.93 (brs, 8H), 3.58 (brs, 2H), 2.81 (s, 3H), 1.31 (t, J = 5.0 Hz, 3H)

### Example 4: N-(3-fluoro-4-((2-(1-methyl-1H-imidazol-4-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide

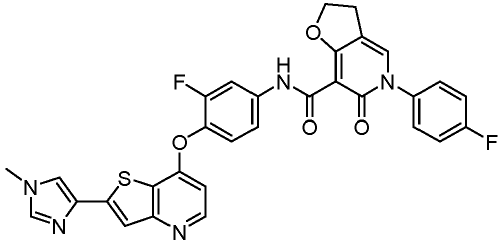

The title compound was synthesized by a method described in Example 8 of Korean Patent Laid-open Publication No. 2021-0015730.

¹H NMR (500 MHz, DMSO-d₆) δ 11.92 (s, 1H), 8.43 (d, J = 5.0 Hz, 1H), 7.99 (d, J = 10.0 Hz, 1H), 7.87 (d, J = 5.0 Hz, 2H), 7.72 (s, 1H), 7.68 (s, 1H), 7.52-7.50 (m, 2H), 7.44-7.36 (m, 4H), 6.59 (d, J = 5.0 Hz, 1H), 4.85 (t, J = 10.0 Hz, 2H), 3.72 (s, 3H), 3.10 (t, J = 10.0 Hz, 2H)

### Example 5: N-(3-fluoro-4-((6-methoxy-7-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide

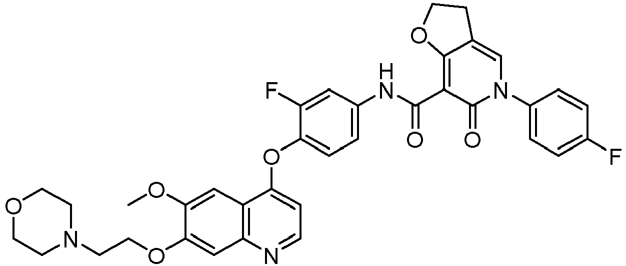

The title compound was synthesized by a method described in Example 78 of Korean Patent Laid-open Publication No. 2021-0015730.

¹H NMR (500 MHz, DMSO-d₆) δ 11.89 (s, 1H), 8.48 (d, J = 5.0 Hz, 1H), 8.00 (d, J = 10.0 Hz, 1H), 7.88 (s, 1H), 7.54-7.49 (m, 3H), 7.45-7.37 (m, 5H), 6.48 (d, J = 5.0 Hz, 1H), 4.85 (t, J = 10.0 Hz, 2H), 4.32 (brs, 2H), 3.95 (s, 3H), 3.63 (brs, 4H), 3.11 (t, J = 10.0 Hz, 2H), 2.52 (m, 2H, partially overlapped with DMSO), 2.50 (m, 4H, overlapped with DMSO)

### Example 6: N-(4-((7-(3-(3-cyanoazetidine-1-yl)propoxy)-6-methoxyquinoline-4-yl)oxy)-3-fluorophenyl)-5-(4-fluorophenyl)-6-oxo-2,3,5,6-tetrahydrofuro[3,2-c]pyridine-7-carboxamide

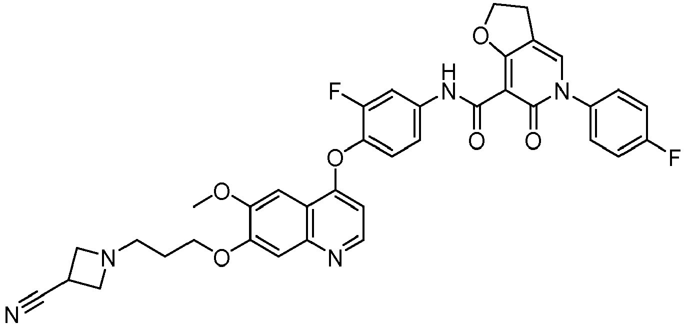

The title compound was synthesized by a method described in Example 88 of Korean Patent Laid-open Publication No. 2021-0015730.

¹H NMR (400 MHz, CDCl₃) δ 12.04 (S, 1H), 8.48 (d, J = 5.2 Hz, 1H), 8.03 (dd, J = 12.4, 2.4 Hz, 1H), 7.57 (s, 1H), 7.40-7.37 (m, 5H), 7.26-7.22 (m, 2H), 7.17 (t, J = 8.8 Hz, 1H), 6.44 (d, J = 5.6 Hz, 1H), 5.01 (t, J = 8.4 Hz, 2H), 4.26 (t, J = 6.4 Hz, 2H), 4.03 (s, 3H), 3.64 (t, J = 11.6 Hz, 4H), 3.20 (t, J = 8.0 Hz, 2H), 2.81 (t, J = 6.8 Hz, 2H), 2.05 (q, J = 6.8 Hz, 3H)

### Positive control 1

N-{4-[(2-amino-3-chloro-4-pyridinyl)oxy]-3-fluorophenyl}-4-ethoxy-1-(4-fluorophenyl)-2-oxo-1,2-dihydro-3-pyridinecarboxamide, corresponding to Compound 1 disclosed in U.S. Patent No. 8,536,200 B2 which is BMS-777607, a well known RON inhibitor, was used as the compound of positive control 1.

### Positive control 2

3-[(R)-1-(2,6-dichloro-3-fluorophenyl)-ethoxy]-5-[1-(piperidine-4-yl-1H-pyrazole-4-yl]-pyridine-2-amine, corresponding to Compound 1 disclosed in PCT International Publication WO 2013/017989 A1 which is crizotinib, a well known anticancer drug, was used as the compound of positive control 2.

### Positive control 3

N-(4-{[6,7-bis(methyloxy)quinoline-4-yl]oxy}phenyl)-N'-(4-fluorophenyl)cyclopropane-1,1-dicarboxamide, corresponding to Compound 1 disclosed in PCT International Publication WO 2014/165786 A1 which is cabozantinib, a well known anticancer drug, was used as the compound of positive control 3.

### Negative control

Dimethyl sulfoxide (DMSO) was used as a compound of a negative control.

### Experimental Example 1. Analysis of efficacy of compound on colorectal cancer cell line with KRAS mutation depending on RON mutation

MTS assay was performed to compare cytotoxicity of the compounds of Example 1, Example 2, Example 3, Example 4, Example 5, Example 6, and positive control 1 on a colorectal cancer cell line, and a cell death rate of the colorectal cancer cell line was analyzed to compare anticancer activity of the compounds.

The MTS assay was performed by seeding each colorectal cell line in a 96-well plate at 1,000 cells per well, and after 24 hours, performing treatment with the compounds of Examples 1 to 6 and positive control 1 at various concentrations (0.000001 µM to 10 µM) in 100 µL of 10% FBS-RPMI medium (WELGENE Inc.) per well. The cells were incubated for 72 hours. Then, 20 µL of MTS solution (Promega) was added thereto, and the cells were incubated for 2 hours. Then, the absorbance was measured at 490 nm. The IC₅₀ values for the compounds of Examples 1 to 6 and positive control 1 were calculated using GraphPad Prism^{™} 5 to investigate cytotoxicity. The compounds of Examples 1 to 6 exhibited high efficiency in most cell lines except for SNUC2A with inactive RON. It was confirmed that the compound of Example 4 among them exhibited high efficiency in the cell lines (HCT8 and DLD-1) which express most mRON or activated RON (pTyr-wtRON) except for SW620. In contrast, the compound of positive control 1 exhibited high IC₅₀ values in most cell lines, and thus it was confirmed that the compound had low response to drugs.

The results of the MTS assay are as shown in Table 1 below:

**[Table 1]**

| Cell line | | SNUC2 A | HCT8 | DLD1 | HCT15 | SW480 | SW620 | T84 |
|---|---|---|---|---|---|---|---|---|
| RON mutation | | negative | wt | wt | △160 | △155 | △155 | △165 |
| KRAS mutation | | G12D | G13D | G13D | G13D | G12V | G12V | G13D |
| MTS IC₅₀ (µM) | Positive control 1 | >10 | 7.32 | 4.72 | 3.24 | 3.54 | 5.00 | 8.40 |
| | Example 1 | 5.78 | 0.42 | 0.24 | 0.23 | 0.34 | 0.32 | 0.35 |
| | Example 2 | 4.44 | 0.26 | 0.21 | 0.22 | 1.25 | 0.37 | 0.21 |
| | Example 3 | 4.60 | 0.48 | 0.30 | 0.39 | 0.66 | 0.26 | 0.22 |
| | Example 4 | 7.53 | 1.20 | 0.66 | 1.27 | 0.67 | >10 | 0.17 |
| | Example 5 | 3.82 | 0.78 | 0.23 | 0.39 | 0.11 | 1.00 | 0.81 |
| | Example 6 | 1.32 | 0.33 | 0.17 | 0.14 | 0.32 | 0.11 | 0.20 |

Based on the results above, to identify the cell death-inducing ability of the compounds of Example 1 and positive control 1, which are representative materials, preparation was performed by seeding respective colorectal cancer cell lines SNU-C2A, HCT8, T84, and SW620 onto 60-mm plates at 3 × 10⁵ cells, and then incubation was performed for 24 hours. Thereafter, treatment with each of Example 1 and positive control 1 was performed at 1 µM and incubation was performed for 48 hours.

In addition, preparation was performed by seeding the cell line SW620 onto a 60-mm plate at 3 × 10⁵ cells, and then incubation was performed for 24 hours. Thereafter, treatment with each of Example 1, positive control 1, positive control 2, and positive control 3 was performed at 1 µM.

After 48 hours, the cells and cell suspensions were collected by treatment with trypsin, resuspended in IX PBS and stained using a trypan blue solution (Sigma, cat# T8154). The stained cells were counted using a hemocytometer. Cell counting was repeated, and the mean and the standard deviation were calculated.

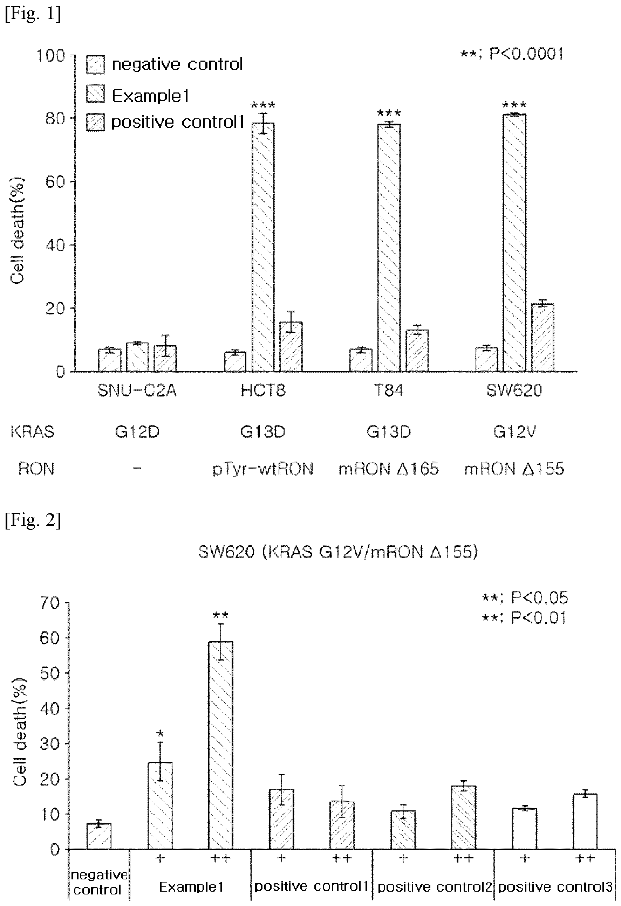

Regarding the KRAS mutant colorectal cancer cell lines, cell death induction efficacy of the compounds of Example 1 and positive control 1 depending on the presence or absence of a RON mutation was checked. As a result, as shown in FIG. 1, it was confirmed that cell-killing efficacy was exhibited by treatment with the compound of Example 1 in cell line HCT8 that expresses RON (pTyr-wtRON) which is wild-type and tyrosine-phosphorylated. In addition, it was confirmed that cell death induction efficacy was exhibited regardless of mutation type when cell lines with a RON mutation were treated with the compound of Example 1. Meanwhile, it was confirmed that the cell line SNUC2A with inactive RON showed no response to the compound of Example 1, and that even the cell lines T84 and SW620 with a RON mutation showed little response to the compound of positive control 1. In addition, it was confirmed that a high cell death rate was observed in the case where the cell line SW620 was treated with the compound of Example 1. On the contrary, as shown in FIG. 2, it was confirmed that cell death was not induced in the cell line SW620 treated with the compound of positive control 1, positive control 2, or positive control 3.

### Experimental Example 2. Analysis of efficacy of compound in colorectal cancer patient-derived cell lines

In colorectal cancer patient-derived cell lines, RON, KRAS, and BRAF mutations were analyzed. The information on each patient is as shown in Table 2 below, and the genotyping result thereof is as shown in Table 3 below:

**[Table 2]**

| Division | Patient Information | | | | | | |
|---|---|---|---|---|---|---|---|
| | Age | Gender | Stage | Grade | Surgery | treatment | Histogenic type |
| WMB-C-P2 | 27 | Female | IV | G2 | Palliative LAR | FOLFIRI #8 Bev/capecita bine #6 | ADENOCARCI NOMA, MD |
| WMB-C-P6 | 53 | Male | IIIB | G2 | RHC | No | ADENOCARCI NOMA, MD |

**[Table 3]**

| Division | Genotype | | |
|---|---|---|---|
| | RON | KRAS | BRAF |
| WMB-C-P2 | Wild | G12V | Wild |
| WMB-C- P6 | Δ160 | G12H | Wild |

RNA was extracted using a Trizol reagent from each colorectal cancer patient-derived cell line, and then subjected to reverse transcription through reverse transcriptase. PCR was performed using primers capable of identifying a RON mutation in the reverse-transcribed sample, and sequencing was performed on each band to identify the mutation. The nucleotide sequences of the primers used are as follows.

**[Table 4]**

| Primer | Nucleotide sequence | SEQ ID NO |
|---|---|---|
| RON d5_6 sense primer | 5′-GAGCTGGTCAGGTCACTAAAC- 3′ | SEQ ID NO: 5 |
| RON d5_6 antisense primer | 5′-CAGACACTCAGTCCCATTGAC- 3′ | SEQ ID NO: 6 |
| RON d11 sense primer | 5'- ATCTGTGGCCAGCATCTAAC-3' | SEQ ID NO: 7 |
| RON d11 antisense primer | 5′- AAAGGCAGCAGGATACCAAG- 3′ | SEQ ID NO: 8 |

Regarding KRAS, analysis was performed using the same method to identify whether KRAS is a wild type or a mutant. In addition, each cell was fixed with 4% paraformaldehyde, and then staining was performed on pTyr-RON according to an immunostaining protocol to identify whether the staining is positive or negative. Then, each cell line was treated with the compound of Example 1 at 1 µM, and staining of cleaved caspase 3 was performed to identify whether the compound exhibits cell death induction efficacy, and a cell death rate was calculated.

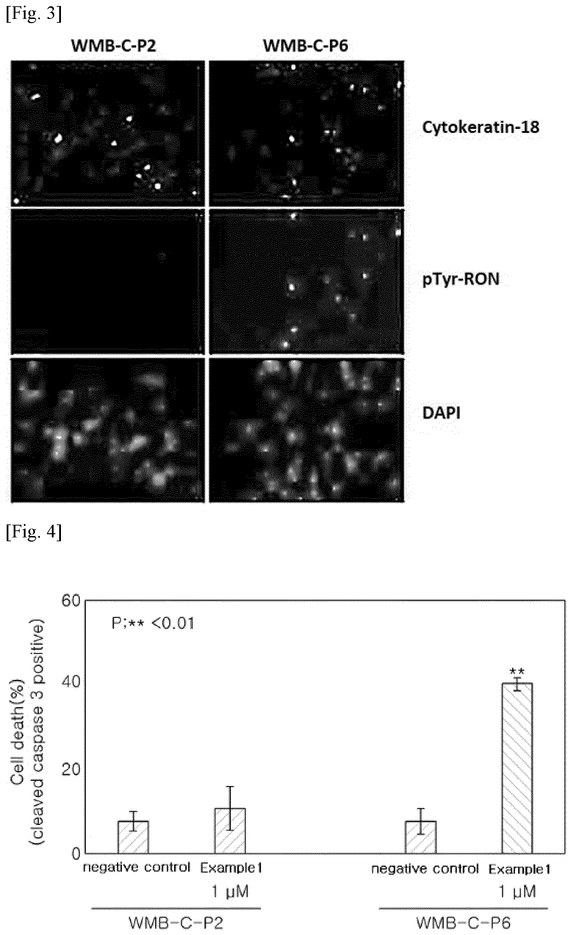

The colorectal cancer patient-derived cell line was analyzed for KRAS and RON mutations, and then the expression of pTyr-wtRON was analyzed through immunofluorescence staining in the case where RON is a wild type (see FIG. 3). As shown in FIG. 4, it was confirmed that the compound of Example 1 exhibited cell death induction efficacy in the cell line expressing a RON mutation or pTyr-wtRON and that the compound of Example 1 did not exhibit cell death induction efficacy in the cell line with inactive RON.

### Experimental Example 3. Analysis of efficacy of compound in cell line SW620-transplanted mouse model

5-Week-old female BALB/c nude mice were purchased and acclimatized for 1 week. Then, human colorectal cancer patient-derived cell line SW620 (KRAS G12V/mRON Δ155) at 5×10⁶ cells/mouse was injected subcutaneously (100 µL) into the right dorsal side of the mice. The compound of Example 1 was orally administered when the tumor reached a size of about 100 mm³. The drug was administered once a day for 4 weeks, and tumor size and mouse body weight were measured twice a week. After completion of the drug administration, the experimental animals were euthanized, and then the tumors were extracted and weighed for comparative analysis.

A portion of the extracted tumor tissue was fixed in 10% formalin and processed into a paraffin block. Then, the block was made into sections. Staining of the slide was performed through immunochemical staining. Another portion of the extracted tumor tissue was subjected to lysis, and then Western blotting was used to identify changes in expression of respective proteins.

After the cell line SW620 (KRAS G12V/mRON Δ155)-transplanted animal model was administered the compound of Example 1, the tumor growth inhibition rate is as shown in Table 5 below:

**[Table 5]**

| | Example 1 |
|---|---|
| Dosage | 30 mg/kg |
| Tumor growth inhibition (TGI, %) | 79.2±2.2 |

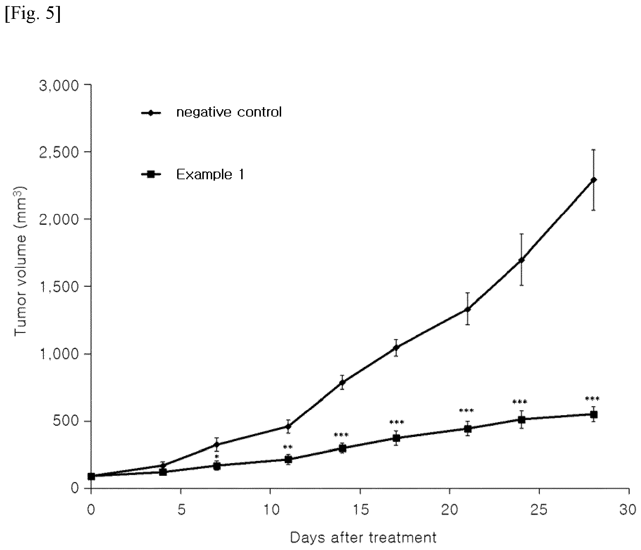

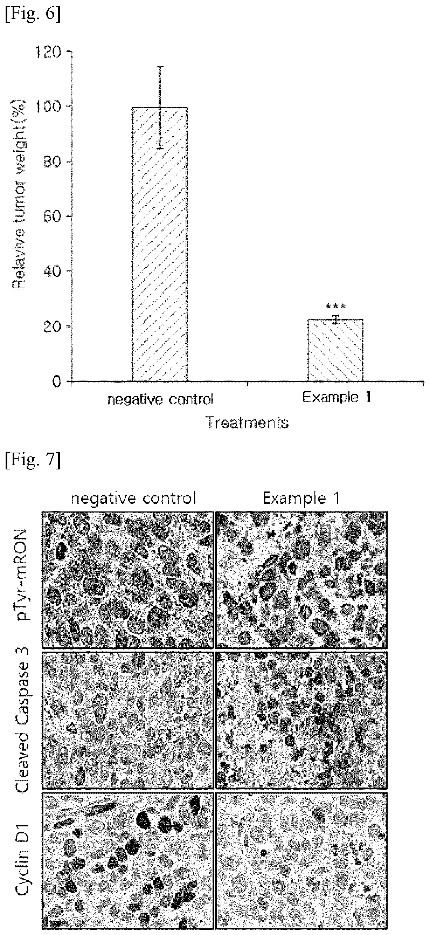

As shown in Table 5, FIG. 5, and FIG. 6, it was confirmed that high tumor growth inhibitory efficacy was exhibited when the compound of Example 1 was administered. In addition, immunochemical staining was performed on the extracted tumor tissue. As a result, as shown in FIG. 7, it was confirmed that decreased expression of pTyr-RON and induced expression of cleaved caspase 3 were exhibited in the mouse model administered the compound of Example 1, and decreased expression of cyclin D1, which is a pharmacodynamic (PD) marker, was exhibited.

### Experimental Example 4. Analysis of efficacy of compound in cell line HCT8-transplanted mouse model

The experiment was conducted in the same method as the procedure of Experimental Example 3, except that human colorectal cancer patient-derived cell line HCT8 (KRAS G13D/pTyr-wtRON(+)) was used in place of the human colorectal cancer patient-derived cell line SW620 (KRAS G12V/mRON Δ155), and the respective compounds of Example 1, positive control 1, and positive control 2 were administered. After the HCT8 (KRAS G13D/pTyr-wtRON(+))-transplanted animal model was administered the respective compounds of Example 1, positive control 1, and positive control 2, the tumor growth inhibition rate is as shown in Table 6 below:

**[Table 6]**

| | Positive control 1 | Positive control 2 | Example 1 |
|---|---|---|---|
| Dosage | 10 mg/kg | 10 mg/kg | 10 mg/kg |
| Tumor growth inhibition (TGI, %) | -7.5 ± 36.3 | 23.2 ± 7.8 | 58.5 ± 9.1 |

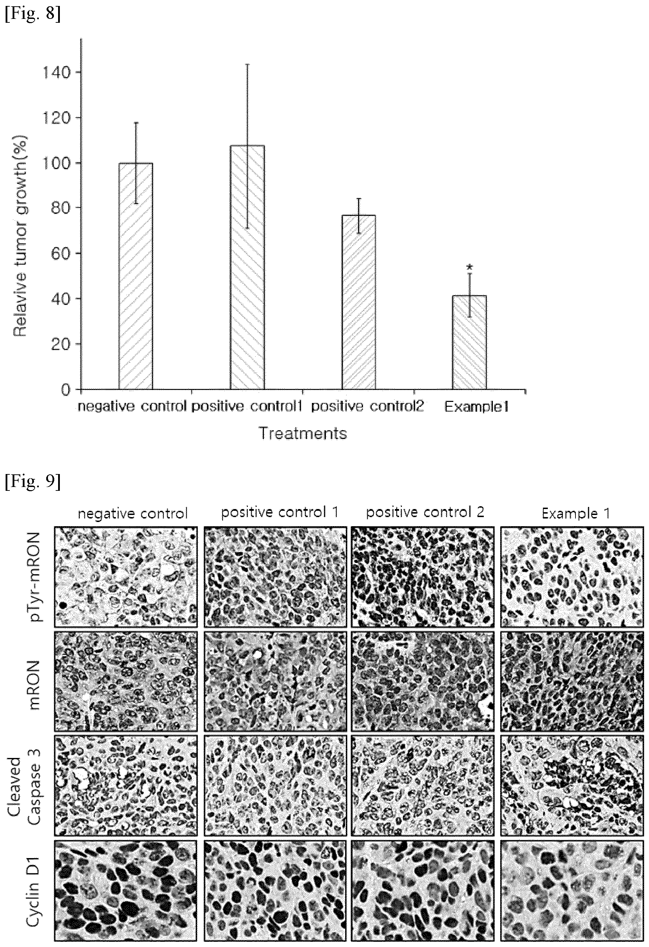

As shown in Table 6 above and FIG. 8, it was confirmed that the animal model administered the compound of Example 1 exhibited high tumor growth inhibitory efficacy as compared with the animal models administered the respective compounds of positive control 1 and positive control 2. In addition, after completion of the drug administration, the tumor tissues were analyzed with immunohistochemical staining. As a result, as shown in FIG. 9, it was confirmed that decreased expression of pTyr-mRON and cyclin D1 was exhibited and induced expression of cleaved caspase 3 was exhibited.

### Experimental Example 5. Analysis of mechanism of action of compound in cell line SW620

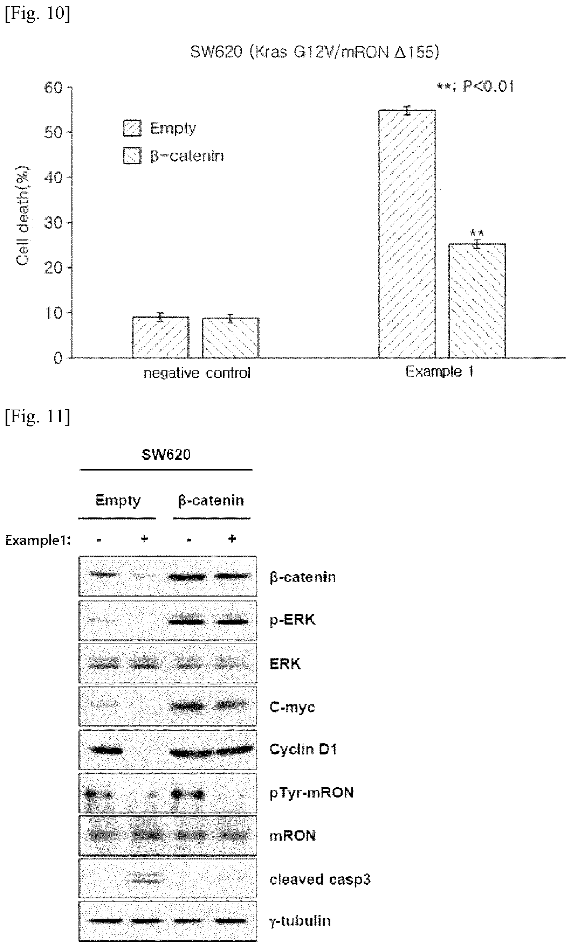

Each of an empty vector and human beta-catenin pcDNA3.1 (Plasmid #16828, Addgene) that is a vector into which β-catenin DNA (X87838.1, GENE ID: 1499) is introduced, was transfected into the human colorectal cancer patient-derived cell line SW620 (KRAS G12V/mRON Δ155), and then treatment with the compound of Example 1 was performed. Subsequently, all cells were collected when 48 hours had elapsed. Cell death induction efficacy was compared and analyzed using a trypan blue exclusion assay. As described above, the cell line SW620 was caused to overexpress β-catenin. Then, treatment with the compound of Example 1 was performed, and cell death induction efficacy was observed. As a result, it was confirmed that cell death induction efficacy of the compound was decreased as shown in FIG. 10.

In addition, as described above, the cell line SW 620 was caused to overexpress β-catenin. Then, the sample treated with the compound of Example 1 was used to identify expressions of β-catenin, p-ERK, ERK, c-myc, cyclin D1, pTyr-mRON, mRON, and cleaved caspase 3 with Western blotting. As a result, as shown in FIG. 11, it was confirmed that expression of pTyr-mRON was decreased by the compound of Example 1 regardless of β-catenin, and that expression of p-ERK, c-myc, and cyclin D1 was decreased in the cell line transfected with the empty vector, but was increased again when β-catenin was overexpressed.

### Experimental Example 6. Analysis of efficacy of compound in cell line HCT15-transplanted mouse model

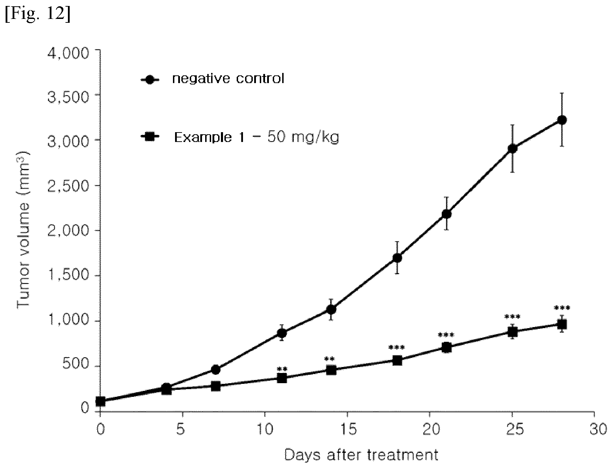

5-Week-old female BALB/c nude mice were purchased and acclimatized for 1 week. Then, human colorectal cancer patient-derived cell line HCT15 (KRAS G13D/mRON Δ160) at 5×10⁶ cells/mouse was injected subcutaneously (100 µL) into the right dorsal side of the mice. The compound of Example 1 was orally administered when the tumor reached a size of about 100 mm³. The drug was administered once a day for 4 weeks, and tumor size and mouse body weight were measured twice a week. As a result, as shown in FIG. 12, it was confirmed that high tumor growth inhibitory efficacy was exhibited in a group administered the compound of Example 1.

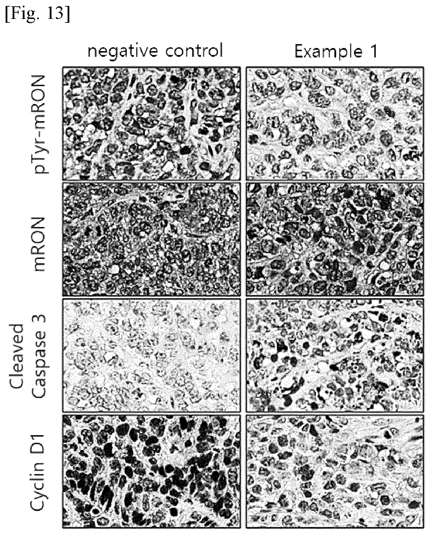

After completion of the drug administration, the tumor tissues were analyzed. As a result, as shown in FIG. 13, it was confirmed that decreased expression of pTyr-mRON was exhibited in the group administered the compound of Example 1 and induced expression of cleaved caspase 3 was exhibited. Additionally, it was confirmed that decreased expression of cyclin D1, which is a PD marker of Example 1, was exhibited in the group administered the compound of Example 1.

## Claims

1. A pharmaceutical composition for preventing or treating cancer with a V-Ki-ras2 Kirsten rat sarcoma viral oncogene homolog (KRAS) mutation and activated recepteur d'origine nantais (RON), the composition comprising, as an active ingredient, a compound of Formula 1 or 2 below or a pharmaceutically acceptable salt thereof,
wherein the KRAS mutation is substitution of glycine, an amino acid residue, at position 13 in the amino acid sequence of SEQ ID NO: 1 with another amino acid, or substitution of glycine, an amino acid residue, at position 12 with another amino acid: 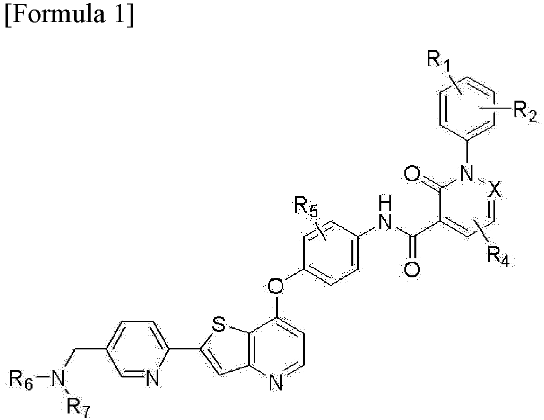 wherein, in Formula 1 above,
R₁ and R₂ are each independently H, halogen, C₁₋₁₀ alkoxy or C₁₋₁₀ haloalkyl;
X is -C(-R₃)= or -N=;
R₃ and R₄ are each independently H, halogen, C₁₋₁₀ alkyl, or C₁₋₁₀ alkoxy;
R₅ is H, halogen, or C₁₋₁₀ alkyl;
R₆ and R₇ form a 4- to 10-membered heterocycle together with the N atom to which they are bonded, or R₆ is -C₂H₄-O-CH₃, and R₇ is H, methyl, or t-butoxycarbonyl; and
the heterocycle, which is mentioned in the definition of substituents in Formula 1 above, optionally further contains one or two heteroatoms selected from the group consisting of N, O, and S, in addition to the N atom to which R₆ and R₇ are bonded, and is unsubstituted or substituted with one or more substituents selected from among halogen and C₁₋₆ alkyl, 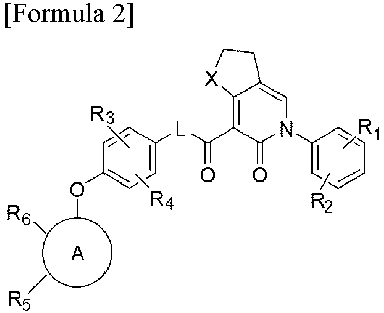 in the formula 2,
L is -NH- or -CH₂-,
R₁ to R₄ are each independently hydrogen, halogen, hydroxy, cyano, C₁₋₄ alkyl, C₁₋₄ alkoxy, C₁₋₄ haloalkyl, C₂₋₄ alkenyl, C₂₋₄ alkynyl, C₃₋₇ cycloalkyl, C₆₋₁₀ aryl, 5- to 9-membered heteroaryl, or 3- to 9-membered heterocycloalkyl,
X is O, S, -CH(-Rx)-, or -N(-Rx)-,
Rx is hydrogen, C₁₋₄ alkyl, C₁₋₄ alkoxy, C₁₋₄ haloalkyl, C₂₋₄ alkenyl, C₂₋₄ alkynyl, C₆₋₁₀ aryl, C₆₋₁₀ aryl-C₁₋₄ alkyl, or 3- to 9-membered heterocycloalkyl,
A is a 3- to 12-membered heterocycle, and
R₅ and R₆ are each independently hydrogen, nitro, amino, halogen, hydroxy, cyano, C₁₋₆ alkyl, C₁₋₆ haloalkyl, C₂₋₆ alkenyl, C₂₋₆ alkynyl, C₁₋₆ alkoxy, amino-C₁₋₆ alkoxy, aminocarbonyl, C₁₋₆ alkylaminocarbonyl, diC₁₋₆ alkylaminocarbonyl, C₁₋₆ alkylcarbonylamino, C₁₋₆ alkylamino, C₁₋₆ alkylamino-C₁₋₆ alkoxy, C₆₋₁₀ aryl, C₆₋₁₀ aryl-C₁₋₄ alkyl, or 5- to 9-membered heteroaryl,
wherein R₅ and R₆ are each independently optionally substituted with C₁₋₆ alkyl; C₁₋₆ alkyl or C₁₋₆ alkylamino-C₁₋₆ alkyl, substituted with any one of C₁₋₆ alkoxy-C₁₋₆ alkylamino, 3- to 9-membered cycloalkyl, and 3- to 9-membered heterocycloalkyl; 3-to 9-membered cycloalkyl; or 3- to 9-membered heterocycloalkyl,
the cycloalkyl or the heterocycloalkyl optionally has one or more substituents selected from the group consisting of halogen, oxo, cyano, hydroxy, hydroxy-C₁₋₆ alkyl, amino, diC₁₋₆ alkylamino, C₁₋₆ alkyl, C₁₋₆ alkoxy, and C₁₋₆ alkoxy-C₁₋₆ alkyl, and
the heterocycle, the heteroaryl, and the heterocycloalkyl, which are mentioned in the definition of substituents in Formula 2 above, each independently contain one or more heteroatoms selected from the group consisting of N, O, and S.

2. The pharmaceutical composition of claim 1, wherein the compound represented by Formula 1 is selected from the group consisting of:
4-ethoxy-N-[3-fluoro-4-({2-[5-(morpholinomethyl)pyridin-2-yl]thieno[3,2-b]pyridin-7-yl}oxy)phenyl]-1-(4-fluorophenyl)-2-oxo-1,2-dihydropyridine-3-carboxamide;
4-ethoxy-N-(3-fluoro-4-{[2-(5-{[(2-methoxyethyl)amino]methyl}pyridin-2-yl)thieno[3,2-b]pyridin-7-yl]oxy}phenyl)-2-oxo-1-phenyl-1,2-dihydropyridine-3-carboxamide; and
4-ethoxy-N-{3-fluoro-4-[(2-{5-[(4-methylpiperazin-1-yl)methyl]pyridin-2-yl}thieno[3,2-b]pyridin-7-yl)oxy]phenyl}-2-oxo-1-phenyl-1,2-dihydropyridine-3-carboxamide.

3. The pharmaceutical composition of claim 1, wherein the compound represented by Formula 2 is selected from the group consisting of:
N-(3-fluoro-4-((2-(1-methyl-1H-imidazol-4-yl)thieno[3,2-b]pyridin-7-yl)oxy)phenyl)-5-(4-fluorophenyl)-6-oxo-2,3,5,6-tetrahydrofuro[3,2-c]pyridine-7-carboxamide;
N-(3-fluoro-4-((6-methoxy-7-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide; and
N-(4-((7-(3-(3-cyanoazetidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide.

4. The pharmaceutical composition of claim 1, wherein the activated RON is a splicing variant of RON gene or tyrosine-phosphorylated RON protein.

5. The pharmaceutical composition of claim 4, wherein the splicing variant of the RON gene is mRONΔ155 in which exons 5, 6, and 11 are deleted, mRONΔ160 in which exons 5 and 6 are deleted, or RONΔ165 in which exon 11 is deleted.

6. The pharmaceutical composition of claim 1, wherein the KRAS mutation is substitution of the glycine at position 12 with any one selected from the group consisting of aspartic acid, valine, cysteine, and histidine.

7. The pharmaceutical composition of claim 1, wherein the KRAS mutation is substitution of the glycine at position 13 with any one selected from the group consisting of aspartic acid, valine, and arginine.

8. The pharmaceutical composition of claim 1, wherein the cancer is selected from the group consisting of breast cancer, lung cancer, stomach cancer, prostate cancer, uterine cancer, ovarian cancer, kidney cancer, pancreatic cancer, liver cancer, colorectal cancer, skin cancer, head and neck cancer, and thyroid cancer.

9. A use of the compound represented by Formula 1 or 2 defined in claim 1 or a pharmaceutically acceptable salt thereof for preventing or treating cancer with a KRAS mutation and activated RON,
wherein the KRAS mutation is substitution of glycine, an amino acid residue, at position 13 in the amino acid sequence of SEQ ID NO: 1 with another amino acid, or substitution of glycine, an amino acid residue, at position 12 with another amino acid.

10. A use of the compound represented by Formula 1 or 2 defined in claim 1 or a pharmaceutically acceptable salt thereof for preparation of a medication for preventing or treating cancer with a KRAS mutation and activated RON,
wherein the KRAS mutation is substitution of glycine, an amino acid residue, at position 13 in the amino acid sequence of SEQ ID NO: 1 with another amino acid, or substitution of glycine, an amino acid residue, at position 12 with another amino acid.

11. A method for preventing or treating cancer with a KRAS mutation and activated RON, the method comprising administering the compound represented by Formula 1 or 2 as defined in claim 1 or a pharmaceutically acceptable salt thereof to a subject in need thereof,
wherein the KRAS mutation is substitution of glycine, an amino acid residue, at position 13 in the amino acid sequence of SEQ ID NO: 1 with another amino acid, or substitution of glycine, an amino acid residue, at position 12 with another amino acid.

12. A method for providing information on an anticancer therapeutic agent, the method comprising:
identifying a KRAS mutation and activated RON in an individual; and
providing information that the compound represented by Formula 1 or 2 defined in claim 1 or a pharmaceutically acceptable salt thereof is suitable for anticancer therapy of the individual when activated RON and a KRAS mutation in which glycine, an amino acid residue, at position 13 in the amino acid sequence of SEQ ID NO: 1 is substituted with another amino acid or glycine, an amino acid residue, at position 12 is substituted with another amino acid, are found.
